# EUROPEAN PATENT APPLICATION

(11) **EP 3 449 977 A1**
(43) Date of publication of application: **06.03.2019**
(21) Application number: 18200582.7
(22) Date of filing: 17.03.2014
(51) Int. Cl.: A61P 9/12, A61K 39/395, G01N 33/53

(54) **METHOD FOR TREATING ECLAMPSIA AND PREECLAMPSIA**

(30) Priority: 15.03.2013 US 201361799438 P
(62) Divisional of application: 14764490.0
(71) Applicant: Velo Bio, LLC, Morrisville, North Carolina 27560 (US)
(72) Inventor: ADAIR, Charles, Signal Mountain, TN Tennessee 37377 (US)
(74) Representative: Henderson, Helen Lee

(57) **Abstract**

Methods for detecting patients with eclampsia or preeclampsia by detecting of EDLF in a patient. Methods for screening patients that may be responsive to anti-digoxin antibody therapy are also described. Systems for detecting EDLF include nanowire biosensors.

## Description

### CROSS-REFERENCE TO RELATED APPLICATIONS

This International Patent Application claims priority to U.S. Provisional Patent Application No. 61/799,438, filed March 15, 2013, the disclosure of which is hereby incorporated by reference in its entirety.

### FIELD OF THE INVENTION

The present invention relates to the diagnosis and treatment of eclampsia and preeclampsia comprising measurement of endogenous digitalis-like factors (EDLFs) levels in pregnant women and the administration of an effective amount of anti-digoxin antibody or binding fragments thereof. The invention also entails treatment and/or prevention of conditions associated with fetal complications during pregnancy, *i.e.,* intraventricular hemorrhage. The invention further comprises techniques for the detection and measurement of EDLFs which provide a measurement of levels of EDLFs as well as an indication whether a patient is EDLF positive. Further provided are techniques for administering an effective amount of anti-digoxin antibody or binding fragments thereof to treat any such conditions.

### BACKGROUND OF THE INVENTION

Preeclampsia (PE) is a leading cause of death among pregnant women. Hypertension and proteinuria in the second half of pregnancy are still used to diagnose this disorder, but generalized edema, hyperuricemia, thrombocytopenia, neurologic changes and/or elevated liver enzymes can also occur and may herald more severe forms of the disease. Roberts Semin Perinatal. 2000;24(1):24-28; Roberts, et al. Am J Obstet Gynecol. 1989; 161 (5): 1200-1204. PE occurs more often in first pregnancies and in women who are obese. Zavalza-Gomez Arch Gynecol Obstet. 2011;283:415-422. Additionally, PE frequently leads to intrauterine growth restriction (IUGR) and is a leading cause of preterm delivery. Ness & Sibai Am J Obstet Gynecol. 2006;195:40-49.

Many abnormalities have been reported in established PE but the relation of each of these abnormalities to the cause or intermediate pathophysiology of PE is still unknown. One abnormality frequently seen, which may precede clinically established disease, is generalized damage to the maternal vascular endothelium. These changes appear weeks earlier in pregnancies destined to develop PE. Roberts, et al. Am J Obstet Gynecol. 1989; 161 (5): 1200-1204. Such injury is accompanied by the release of markers of endothelial dysfunction, including pro-inflammatory cytokines, reactive oxygen species, cell surface adhesion molecules, and locally acting modulators of vascular tone, secondary to the original damage. LaMarca, et al. Current Hypertension Reports. 2007; 9 (6): 480-485. Since the causes of PE are incompletely known, there is no direct way to test if or when PE will occur, and no effective treatment to prevent or treat PE. When it is severe, even if it occurs early in the pregnancy, the only effective treatment is to deliver the fetus in order to protect the mother.

As to the earliest physiologic events that lead to PE, there is evidence to suggest that PE begins as a pregnancy complicated by early placental dysfunction. Sankaralingam, et al. Expert Rev in Mol Med. 2006;8: 1-20. Current thinking is that PE is, at least in part, due to an incomplete adaptation of maternal spiral arteries that supply blood, with its oxygen and nutrients, to the fetal- placental unit. This is thought to result in under perfusion, hypoxia, placental damage with local release of reactive oxygen species and pro-inflammatory mediators, including activation of leukocytes and platelets, increasing circulating cytokine levels, e.g. tumor necrosis factor α (TNF α), interferon-γ and interleukin-6. Granger, et al. Microcirculation. 2002; 9 (3): 147-160; Soleymanlou, et al. J Clin Endocrinol Metab. 2005; 90 (7): 4299-4308. These in turn may lead to more generalized effects on placental, fetal, and maternal endothelial function, causing a more exaggerated inflammatory response with evidence of broader maternal, and perhaps fetal, oxidative stress potentially accounting for the endothelial damage evident in the mother with clinical disease.

An additional abnormality found in women with PE is an elevation in the circulating level of endogenous "digitalis-like" factors (EDLFs) that appear to be native inhibitors of [Na⁺,K⁺]ATPase (also termed the sodium pump, SP). Graves & Williams J Clin Endocrinol Metab. 1984; 59:1070-4; Valdes, et al. Prog Clin Biol Res. 1985; 192:229-32; Graves Hypertens. 1987; 10(S Pt 2):I-84-6; Graves, et al. Am J Hypertens. 1995; 8:5-11. Additionally, these factors have been shown to crossreact with digoxin, ouabain and other cardiac glycoside antibodies, suggesting strongly that EDLFs are compounds structurally related to digoxin, ouabain, bufalin, proscillaridin A and/or marinobufagenin. Hamlyn, et al. Proc Natl Acad Sci USA. 1991; 88: 6259-6263; Goto & Yamada Clin Exp Hypertens._1998; 20: 551-56. However, the exact structure of the circulating factor in PE is unknown. EDLFs which share biological and immunological properties with known cardiotonic drugs, such as digoxin, have been found in a number of tissues and body fluids of animals and humans. Increased levels of EDLF may be a causative factor in the pathogenesis of essential, some secondary and experimental hypertension. Glatter, et al. Am J Hypertens, 1994; 7:1016-25; Krep, et al. Am J Hypertens. 1995; 8:921-7; Krep, et al. Am J Hypertens. 1995; 9:39-46.

Digibind and Digifab are each a commercially available Fab fragment derived from polyclonal anti-digoxin antibodies raised in sheep. Digibind and Digifab (referred to as DigoxinAB) are used for treatment of digoxin overdose and accompanying toxicity by binding digoxin and making digoxin unavailable for binding to the [Na⁺, K⁺]ATPase. Smith, et al. (1982) N Engl J Med. 307: 1357-62. The DigoxinAB -digoxin complex accumulates in the blood and is excreted by the kidney. The net effect is to shift the equilibrium away from binding of digoxin to its receptors in the body, reversing its effects. In experimental models of hypertension with elevated EDLF levels, DigoxinAB has been demonstrated to lower blood pressure, providing evidence that the antibody crossreacts with and inactivates EDLF. This is supported by a much larger *in vitro* literature. Krep, et al. (1995) Am J Hypertens. 8: 921-7; Krep, et al. (1995) Am J Hypertens. 9: 39-46.

Previous studies have reported that EDLF was structurally the steroidal glycoside digoxin or an analogue having an unsaturated lactone ring that it is synthesized by the isoprenoid/steroid pathway, a very complex route that involves multiple steps. For this research key intermediates of an endogenous digoxin synthetic pathway were of interest. Previous studies of steroid synthesis have demonstrated that ketoconazole is a general inhibitor of cytochrome P450 dependent enzymes, which block gonadal and adrenal steroidogenesis by inhibiting several cytochrome P-450-dependent enzymes. Loose, et al. (1983) J. Clin. Invest. 71: 1495-1499; Miossec, et al. (1997) Ann Endocrinol (Paris) 58: 494-502. It also inhibits cholesterol synthesis in humans by blocking the conversion of methyl sterols to cholesterol. Kraemer, et al. (1986) J Pharmacol Exp Ther. 238: 905-911.

Currently, treatment of eclampsia and preeclampsia is frustrated by the lack of an diagnostic method to identify women with eclampsia, preeclampsia, or symptoms thereof that may respond to anti-digoxin antibody or binding fragments thereof, therapy. Thus, there exists a need in the art for a diagnostic method and concurrent anti-digoxin antibody or binding fragments thereof therapy for women with eclampsia, preeclampsia, or symptoms thereof, *i.e.* a theranostic test to predict which preeclamptic and/or eclamptic women will benefit from treatment with DigoxinAB.

### SUMMARY OF THE INVENTION

In one embodiment, the method of administering digoxin immune Fab (DIF) to treat eclampsia or preeclampsia may comprise: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.

In one embodiment, the method of administering digoxin immune Fab (DIF) to treat a gravid human patient exhibiting at least one symptom of gestational hypertension, preeclampsia, eclampsia, or intrauterine growth restriction may comprise: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.

In one embodiment, the method of treating a gravid human patient exhibiting at least one symptom of gestational hypertension, preeclampsia, eclampsia, or intrauterine growth restriction may comprise: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.

In one embodiment, the method of administering digoxin immune Fab (DIF) to a patient to prevent intraventricular hemorrhage (IVH) in the neonate of the patient may comprise:(a) conducting a digoxin-immune antibody assay on the patient; (b) determining whether the patient is EDLF positive based on assay; and (c) administering digoxin immune Fab (DIF) to patient if the patient is determined to be EDLF positive.

In one embodiment, the method of administering digoxin immune Fab (DIF) to treat intraventricular hemorrhage may comprise:(a) conducting a digoxin-immune antibody assay on a gravid human patient whose fetus may develop IVH as a result of being delivered prematurely (before 40 weeks gestation); (b) determining whether the patient is EDLF positive based on assay; and (c) administering digoxin immune Fab (DIF) to patient if the patient is determined to be EDLF positive.

In one embodiment, the method of administering an anti-digoxin antibody or antigen-binding fragment thereof to prevent intraventricular hemorrhage (IVH) in the neonate of the patient may comprise: (a) conducting a digoxin-immune antibody assay on a gravid human patient whose fetus may develop IVH as a result of being delivered prematurely (before 40 weeks gestation); (b) determining whether the patient is EDLF positive based on assay; and (c) administering the anti-digoxin antibody or antigen-binding fragment thereof to patient if the patient is determined to be EDLF positive.

In one embodiment, the method of administering an anti-digoxin antibody or antigen-binding fragment thereof to treat fetal complications associated with premature birth, including may comprise:(a) conducting a digoxin-immune antibody assay on a gravid human patient whose fetus may be delivered prematurely (before 40 weeks gestation); (b) determining whether the patient is EDLF positive based on assay; and (c) administering the anti-digoxin antibody or antigen-binding fragment thereof to patient if the patient is determined to be EDLF positive.

In another embodiment, the fetal complications associated with premature birth may include IVH or NEC.

In one embodiment, the method of extending pregnancy in a gravid human patient exhibiting at least one symptom of gestational hypertension, preeclampsia, eclampsia, or intrauterine growth restriction may comprise: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.

In one embodiment, the method for treating a patient at risk for eclampsia or preeclampsia may comprise: (a) obtaining a sample from a patient at risk for eclampsia or preeclampsia; (b) contacting said sample with an anti-EDLF antibody or antibody fragment thereof; (c) detecting the presence of an anti-EDLF antibody or antibody fragment-EDLF complex, wherein the presence of said EDLF is indicative of eclampsia or preeclampsia; and (d) administering digoxin immune Fab (DIF) to patient if the patient is determined to be EDLF positive.

In one embodiment, the method for treating a patient whose fetus and/or neonate is at risk for IVH may comprise: (a) obtaining a sample from a patient; (b) contacting said sample with an anti-EDLF antibody or antibody fragment thereof; (c) detecting the presence of an anti-EDLF antibody or antibody fragment-EDLF complex, wherein the presence of said EDLF is indicative of eclampsia or preeclampsia; and (d) administering digoxin immune Fab (DIF) to patient if the patient is determined to be EDLF positive.

In one embodiment, the method for screening patients for responsiveness to anti-digoxin therapy for eclampsia or preeclampsia:(a) obtaining a sample from a patient at risk for eclampsia or preeclampsia; (b) assaying for the presence of EDLF; (c) determining the EDLF level; (d) administering an anti-digoxin antibody or antibody fragment thereof to said patient if said EDLF level is over 100 nm EDLF.

In one embodiment, the method for screening patients for responsiveness to anti-digoxin therapy for gestational hypertension, preeclampsia, eclampsia, or intrauterine growth restriction may comprise:(a) obtaining a sample from a patient suffering from gestational hypertension, preeclampsia, eclampsia, or intrauterine growth restriction; (b) assaying for the presence of EDLF; (c) administering an anti-digoxin antibody or antibody fragment thereof to said patient if the patient is determined to be EDLF positive.

In one embodiment, the method for screening patients for responsiveness to anti-digoxin therapy for gestational hypertension, preeclampsia, eclampsia, or intrauterine growth restriction may comprise: (a) obtaining a sample from a patient at risk for gestational hypertension, preeclampsia, eclampsia, or intrauterine growth restriction; (b) assaying for the presence of EDLF; (c) administering an anti-digoxin antibody or antibody fragment thereof to said patient if the patient is determined to be EDLF positive.

In one embodiment, the method for screening patients for responsiveness to anti-digoxin therapy for gestational hypertension, preeclampsia, eclampsia, or intrauterine growth restriction may comprise:(a) obtaining a sample from a patient at risk for gestational hypertension, preeclampsia, eclampsia, or intrauterine growth restriction; (b) assaying for the presence of EDLF;(c) determining the EDLF level; (d) administering an anti-digoxin antibody or antibody fragment thereof to said patient if said EDLF level is over 100 nm EDLF.

In one embodiment, the method of administering anti-digoxin antibody or antigen-binding fragment thereof to treat intraventricular hemorrhage may comprise: (a) conducting a digoxin-immune antibody assay on a patient suffering from intraventricular hemorrhage; (b) determining whether the patient is EDLF positive based on assay; and (c) administering the anti-digoxin antibody or antigen-binding fragment thereof to patient if the patient is determined to be EDLF positive.

In one embodiment, the method for treating intraventricular hemorrhage may comprise: (a) obtaining a sample from a patient whose fetus is at risk for intraventricular hemorrhage; (b) assaying for the presence of EDLF; (c) determining the EDLF level; (d) administering an anti-digoxin antibody or antibody fragment thereof to said patient if said EDLF level is over 100 nm EDLF.

In one embodiment, the method for screening patients for responsiveness to anti-digoxin therapy for intraventricular hemorrhage: (a) obtaining a sample from a patient at risk for intraventricular hemorrhage; (b) assaying for the presence of EDLF; (c) determining the EDLF level; (d) administering an anti-digoxin antibody or antibody fragment thereof to said patient if said EDLF level is over 100 nm EDLF.

In one embodiment, the method for treating a patient at risk for intraventricular hemorrhage may comprise: (a) obtaining a sample from a patient at risk for intraventricular hemorrhage; (b) contacting said sample with an anti-EDLF antibody or antibody fragment thereof; and (c) detecting the presence of an anti-EDLF antibody or antibody fragment-EDLF complex, wherein the presence of said EDLF is indicative of intraventricular hemorrhage.

In one embodiment, the method of administering digoxin immune Fab (DIF) to treat eclampsia or preeclampsia may comprise: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.

In one embodiment, the method of administering digoxin immune Fab (DIF) to treat a gravid human patient exhibiting at least one symptom of gestational hypertension, preeclampsia, eclampsia, or intrauterine growth restriction may comprise: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.

In one embodiment, the method of treating a gravid human patient exhibiting at least one symptom of gestational hypertension, preeclampsia, eclampsia, or intrauterine growth restriction may comprise: (a) conducting a digoxin-immune antibody immunoassay on a patient suffering from eclampsia or preeclampsia; (b) determining whether the patient is EDLF positive based on immunoassay; and (c) administering digoxin immune Fab (DIF) to patient if the patient is determined to be EDLF positive.

In one embodiment, the method of administering digoxin immune Fab (DIF) to a patient to prevent intraventricular hemorrhage (IVH) in the neonate of the patient may comprise:(a) conducting a digoxin-immune antibody immunoassay on the patient; (b) determining whether the patient is EDLF positive based on immunoassay; and (c) administering digoxin immune Fab (DIF) to patient if the patient is determined to be EDLF positive.

In one embodiment, the method of administering digoxin immune Fab (DIF) to treat intraventricular hemorrhage may comprise:(a) conducting a digoxin-immune antibody immunoassay on a gravid human patient whose fetus may develop IVH as a result of being delivered prematurely (before 40 weeks gestation); (b) determining whether the patient is EDLF positive based on immunoassay; and (c) administering digoxin immune Fab (DIF) to patient if the patient is determined to be EDLF positive.

In one embodiment, the method of administering an anti-digoxin antibody or antigen-binding fragment thereof to prevent intraventricular hemorrhage (IVH) in the neonate of the patient may comprise: (a) conducting a digoxin-immune antibody immunoassay on a gravid human patient whose fetus may develop IVH as a result of being delivered prematurely (before 40 weeks gestation); (b) determining whether the patient is EDLF positive based on immunoassay; and (c) administering the anti-digoxin antibody or antigen-binding fragment thereof to patient if the patient is determined to be EDLF positive. In another embodiment, the fetus may be delivered before 40 weeks of gestation.

In one embodiment, the method of administering an anti-digoxin antibody or antigen-binding fragment thereof to treat fetal complications associated with premature birth, including may comprise: (a) conducting a digoxin-immune antibody immunoassay on a gravid human patient whose fetus may be delivered prematurely (before 40 weeks gestation); (b) determining whether the patient is EDLF positive based on immunoassay; and (c) administering the anti-digoxin antibody or antigen-binding fragment thereof to patient if the patient is determined to be EDLF positive.

In one embodiment, the method of administering digoxin immune Fab (DIF) to treat eclampsia or preeclampsia may comprise: (a) conducting a digoxin-immune antibody immunoassay on a patient suffering from eclampsia or preeclampsia; (b) determining whether the patient is EDLF positive based on immunoassay; and (c) administering digoxin immune Fab (DIF) to patient if the patient is determined to be EDLF positive.

In one embodiment, the method of administering digoxin immune Fab (DIF) to treat a gravid human patient exhibiting at least one symptom of gestational hypertension, preeclampsia, eclampsia, or intrauterine growth restriction may comprise: (a) conducting a digoxin-immune antibody immunoassay on a patient suffering from eclampsia or preeclampsia; (b) determining whether the patient is EDLF positive based on immunoassay; and (c) administering digoxin immune Fab (DIF) to patient if the patient is determined to be EDLF positive.

In another embodiment, the fetal complications associated with premature birth may include IVH or NEC.

In one embodiment, the method of extending pregnancy in a gravid human patient exhibiting at least one symptom of gestational hypertension, preeclampsia, eclampsia, or intrauterine growth restriction may comprise: (a) conducting a digoxin-immune antibody immunoassay on a patient suffering from eclampsia or preeclampsia; (b) determining whether the patient is EDLF positive based on immunoassay; and (c) administering digoxin immune Fab (DIF) to patient if the patient is determined to be EDLF positive.

In another embodiment, fragment may be a Fab, Fab', F(ab')2, Fv, CDR, paratope, or portion of an antibody that is capable of binding the antigen.

In another embodiment, anti-digoxin antibody may be chimeric, humanized, anti-idiotypic, single-chain, bifunctional, or co-specific.

In another embodiment, anti-digoxin antibody or antigen-binding fragment may be conjugated to a label. In another embodiment, label may be a chemiluminescent label, paramagnetic label, an MRI contrast agent, fluorescent label, bioluminescent label, or radioactive label. In another embodiment, paramagnetic label may be aluminum, manganese, platinum, oxygen, lanthanum, lutetium, scandium, yttrium, or gallium.

In another embodiment, anti-digoxin antibody may be attached to a solid support. In another embodiment, solid phase support may be a bead, test tube, sheet, culture dish, nanowire, or test strip. In a further embodiment, the solid support may be an array.

In one embodiment, a silicon nanowire biosensor may comprise an immobilized anti-digoxin antibody, optionally an anti-digoxin Fab antibody fragment. In a further embodiment, the fragment may be a Fab, Fab', F(ab')2, Fv, CDR, paratope, or portion of an antibody that is capable of binding the antigen. In another embodiment, the antibody may be chimeric, humanized, anti-idiotypic, single-chain, bifunctional, or co-specific. In another embodiment, the biosensor may have a sensativity of at least about 100 nM of digoxin in a biological sample.

In one embodiment, the method of detecting EDLF may comprise contacting a biological sample with a nanowire biosensor comprising an immobilized anti-digoxin antibody, optionally an anti-digoxin Fab antibody fragment, and assaying for the presence of EDLF.

In one embodiment, a method for screening patients for responsiveness to anti-digoxin therapy for eclampsia or preeclampsia may comprise (a) obtaining a sample from a patient at risk for eclampsia or preeclampsia, optionally a blood sample; (b) assaying for the presence of EDLF comprising contacting said biological sample with a nanowire biosensor; (c) determining the EDLF level, wherein an EDLF level above about 100 nM is indicative of responsiveness to anti-digoxin therapy for eclampsia or preeclampsia.

In one embodiment, a method for screening patients for responsiveness to anti-digoxin therapy for gestational hypertension, preeclampsia, eclampsia, or intrauterine growth restriction may comprise (a) obtaining a sample from a patient at risk for gestational hypertension, preeclampsia, eclampsia, or intrauterine growth restriction, optionally a blood sample; (b) assaying for the presence of EDLF comprising contacting said biological sample with a nanowire biosensor; (c) determining the EDLF level; wherein an EDLF level over 100 nM is indicative of responsiveness to anti-digoxin therapy for gestational hypertension, preeclampsia, eclampsia, or intrauterine growth restriction.

In one embodiment, a method for screening patients for eclampsia or preeclampsia may comprise (a) obtaining a sample from a patient at risk for eclampsia or preeclampsia, optionally a blood sample; (b) assaying for the presence of EDLF comprising contacting said biological sample with a nanowire biosensor; (c) determining the EDLF level; wherein an EDLF level above 100 nM is indicative of eclampsia or preeclampsia.

In another embodiment, the sample may be a blood, serum, plasma, or placenta sample.

In another embodiment, the anti-digoxin antibody assay may be a Western blot, radioimmunoassay, ELISA (enzyme linked immunosorbent assay), "sandwich" immunoassay, immunoprecipitation assay, precipitation reaction, gel diffusion precipitation reaction, immunodiffusion assay, agglutination assay, complement-fixation assay, immunohistochemical assay, fluorescent immunoassay, a protein A immunoassay, radioimmunoassay, or a combination thereof.

In another embodiment, the EDLF level may be over 100 nM EDLF.

In another embodiment, the administered dosage of digoxin antibody may be at least than 0.006 mg digoxin binding capacity/Kg. In another embodiment, the dosage may be administered over a period of six hours or less.

In one embodiment, the method may further comprise administration of an anti-digoxin antibody, optionally an anti-digoxin immune Fab. In another embodiment, the method may further comprise administration of subsequent dosages of anti-digoxin antibody, optionally an anti-digoxin immune Fab. In another embodiment, the method may further comprise administering a therapeutically effective amount of corticosteroid. In another embodiment, the method may further comprise administration of subsequent dosages of anti-digoxin antibody, optionally an anti-digoxin immune Fab.

In another embodiment, method may further comprise administering a therapeutically effective amount of an antihypertensive drug. In another embodiment, the antihypertensive drug may be labetalol, altenolol, nifedipine, 1-methyldopa, hydralazine, methyldopa (Aldomet), labetalol (Normodyne or Trandate), clonidine (Catapres), or combinations thereof.

In another embodiment, the method may further comprise administering a therapeutically effective amount of magnesium sulfate or phenytoin. In another embodiment, the digoxin immune Fab may be ovine digoxin immune Fab. In another embodiment, the dose may be no more than approximately 10.0 mg. In another embodiment, the dose may be no more than approximately 5.0 mg. In another embodiment, the dose may be in the range between approximately 0.01 to 1.0 mg. In another embodiment, the dose may be in the range between approximately 0.01 mg to 0.5 mg.

### BRIEF DESCRIPTION OF THE DRAWINGS

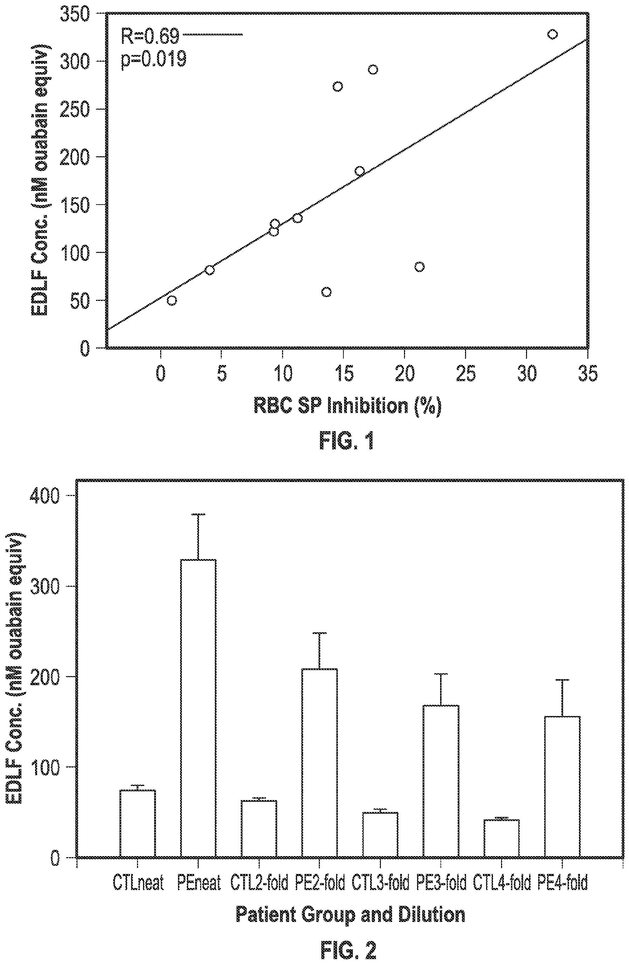

Figure 1: Conditioned media taken from normal human placentas was assayed for EDLF by RIA (y axis) and also for its ability to inhibit the SP in human red cells (x-axis). There was good correlation between the two assays for the several cultured media assayed (R=0.69, p=0.019).
Figure 2 depicts a comparison of the EDLF concentration in protein-free placental homogenates from women with preeclampsia (n=8, PE) versus women with uncomplicated pregnancies (n=8, CTL) by radioimmunoassay. Differences were statistically significant for undiluted homogenate (neat, p=0.0002) or with sequential dilutions (1:2 dilution p=0.002, 1:3 dilution p=0.002, 1:4 dilution p=0.02)

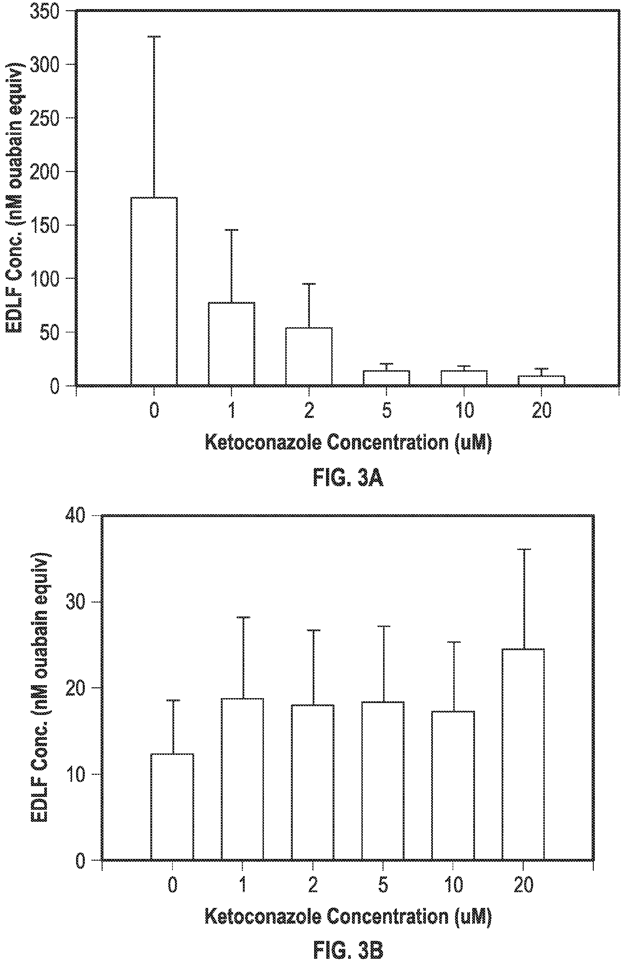

Figures 3A-3B depicts the effect of ketoconazole on placental EDLF production. Human placenta was dissected into fetal and maternal tissues. These were incubated in buffered culture media for 48 hours in the absence or presence of graded concentrations of ketoconazole. The culture media was collected and the amount of EDLF released from the tissue was measured using an RIA. The fetal side data (Panel A) showed a significant and progressive decrease in EDLF production with higher ketoconazole concentration (n=5, p<0.001, ANOVA, EDLF values were significantly lower at all concentrations 2 µM or higher versus the control, Dunnet's test). The maternal side data (Panel B) show little change in EDLF released in response to ketoconazole (n=5, p<0.51, ANOVA).

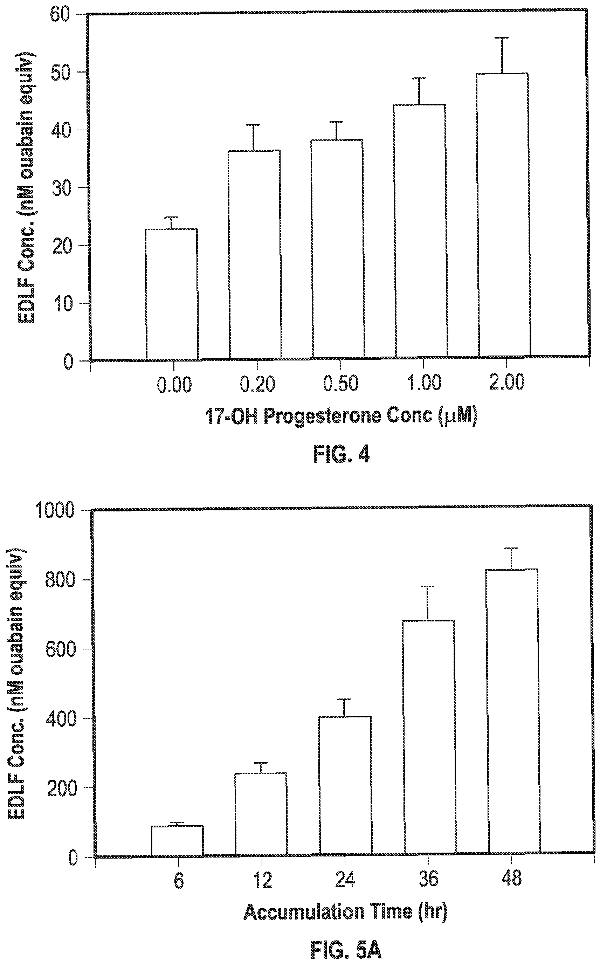

Figure 4 depicts the effect of 17 OH-progesterone on human placental EDLF production. Freshly collected human placenta was dissected and incubated in buffered culture media for 48 hours in the absence or presence of graded concentrations of 17-hydroxyprogesterone. The culture media was collected and the amount of EDLF released from the tissue was measured using an RIA. The data show a progressive increase in EDLF production with higher 17-hydroxyprogesterone concentration (n=6, p=0.003).
Figures 5A-5B depicts the effect of 17 OH-progesterone on human placental EDLF production. Freshly collected human placenta was dissected and incubated in buffered culture media for 48 hours in the absence or presence of graded concentrations of 17-hydroxyprogesterone. The culture media was collected and the amount of EDLF released from the tissue was measured using an RIA. The data show a progressive increase in EDLF production with higher 17-hydroxyprogesterone concentration (n=6, p=0.003).

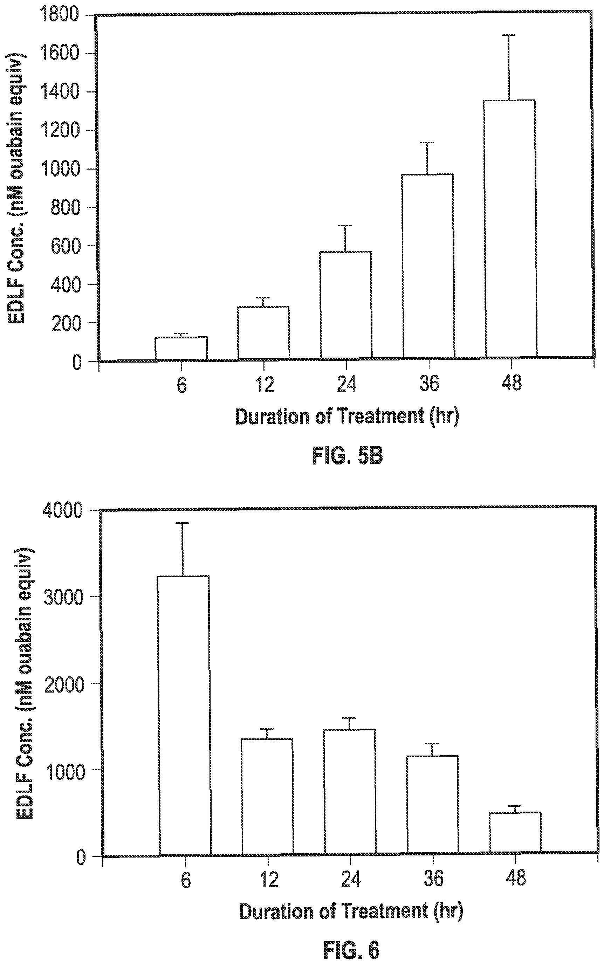

Figure 6 depicts the effects of pregnenolone on human placental EDLF production. Freshly cultured human placenta was exposed to 2 µM pregnenolone for varying periods of time. Placenta exposed to pregnenolone showed marked and progressive reductions in EDLF release (ANOVA, p<0.001, with all subsequent values being reduced compared with the 6 hour value, p<0.05, Dunnett's test).

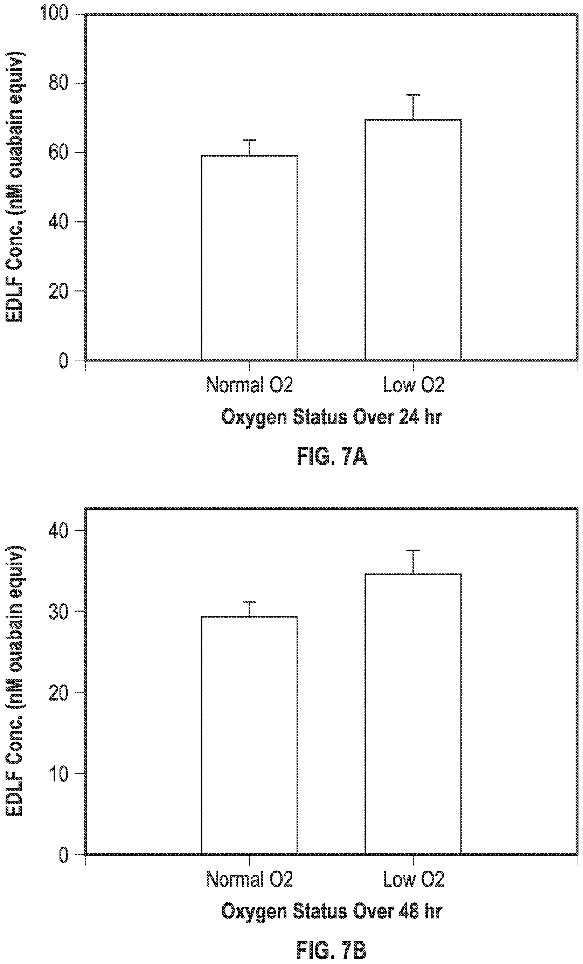

Figures 7A-7B depicts the effect of hypoxia on human placental EDLF production. Freshly collected human placenta was dissected and incubated in buffered culture media under normoxic and hypoxic conditions for 24 hours (Panel A) or 48 hours (Panel B). The culture media was collected and the amount of EDLF released from the tissue was measured using an RIA. The data showed increased EDLF released in response to low O₂ tension at both 24 hours (n=6, p=0.027, Wilcoxon) and 48 hours (n=6, p=0.027, Wilcoxon).

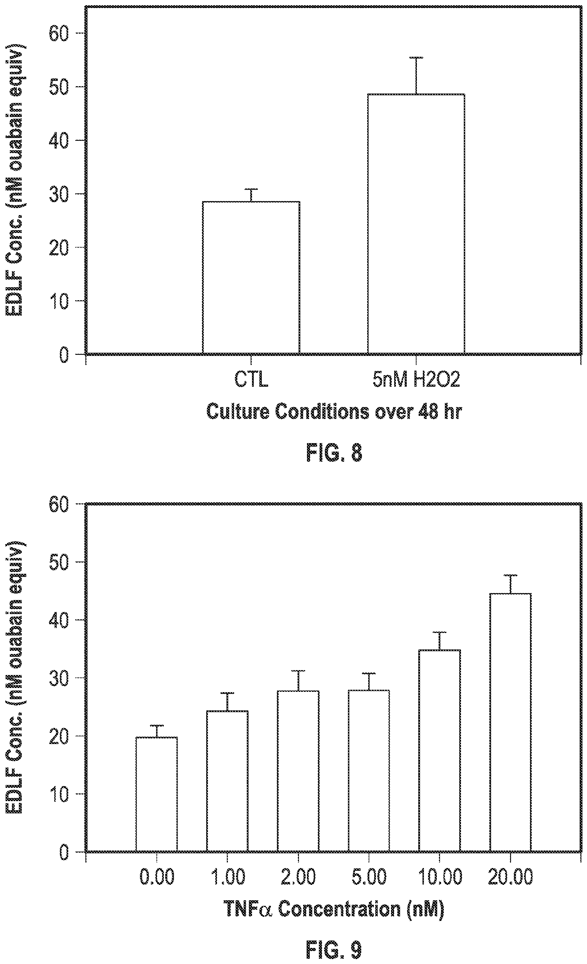

Figure 8 depicts the effect of H₂O₂ on human placental EDLF production. Freshly collected human placenta was cultured for 48 hours in the absence or presence of 5 nM of hydrogen peroxide. The culture media was collected and the EDLF released measured using an RIA. The data show increased EDLF levels released in response to 5 nM H₂O₂ (n=5, p=0.009).
Figure 9 depicts the effect of TNFα on human placental EDLF production. Freshly collected human placenta was incubated for 48 hours in the absence or presence of graded concentrations of TNFα. The culture media was collected and EDLF released from the tissue was measured by RIA. The data show a progressive increase in EDLF production with higher TNFα concentration (n=6, p<0.001).

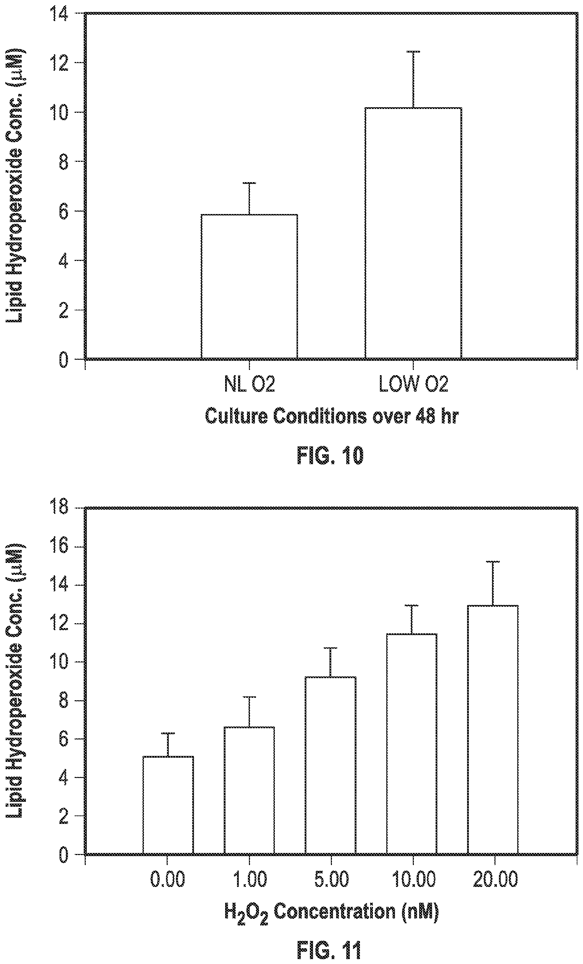

Figure 10 depicts the effect of hypoxia on placental ROS release. Human placenta was incubated in culture media under normoxic and hypoxic conditions for 48 hr. The culture media was collected and the amount of lipid hydroperoxide (LPO) released from the tissue was measured using an ELISA. The data showed increased LPO amount released in response to low O₂ tension (n=6, p=0.01).
Figure 11 depicts the effect of H₂O₂ on placental ROS release. Human placenta was dissected and incubated in buffered culture for 48 hours in the absence or presence of graded concentrations of hydrogen peroxide. The culture media was collected and the amount of lipid hydroperoxide (LPO) released from the tissue was measured by ELISA. The data showed a progressive increase in LPO levels with higher H₂O₂ concentration (n=5, p=0.017, ANOVA).

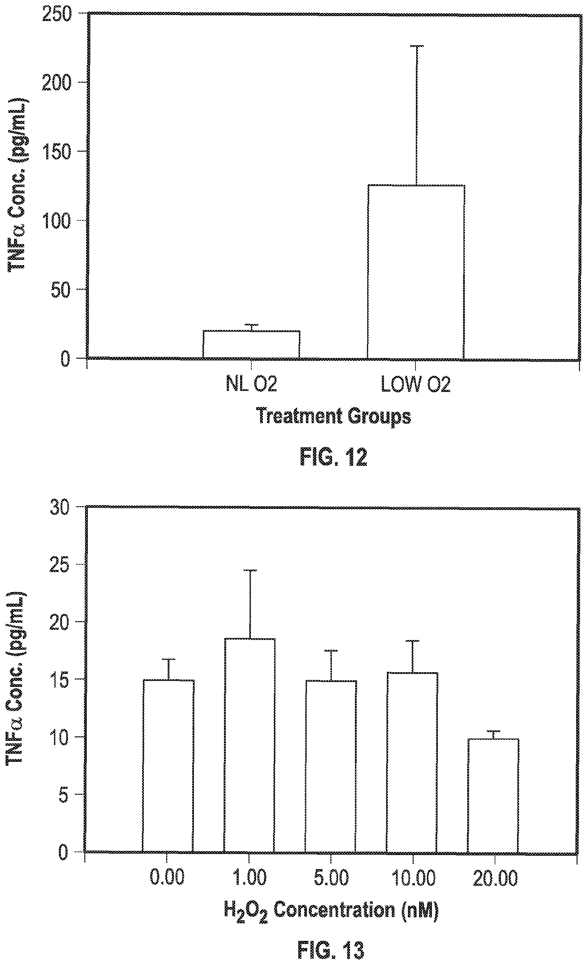

Figure 12 depicts the effect of hypoxia on placental TNFα release. Human placenta was incubated in culture media under normoxic and hypoxia conditions for 48 hr. The culture media was collected and the amount of TNFα released from the tissue was measured by ELISA. The data show increased TNFα amount released in response to low O₂ tension (n=6, p=0.03, Wilcoxon).
Figure 13 depicts the effect of H₂O₂ on placental TNFα release. Human placenta was incubated in buffered culture for 48 hours in the absence or presence of graded concentrations of hydrogen peroxide. The culture media was collected and the amount of lipid TNFα released from the tissue was measured using by ELISA. There was no change (n=5, p=0.91).

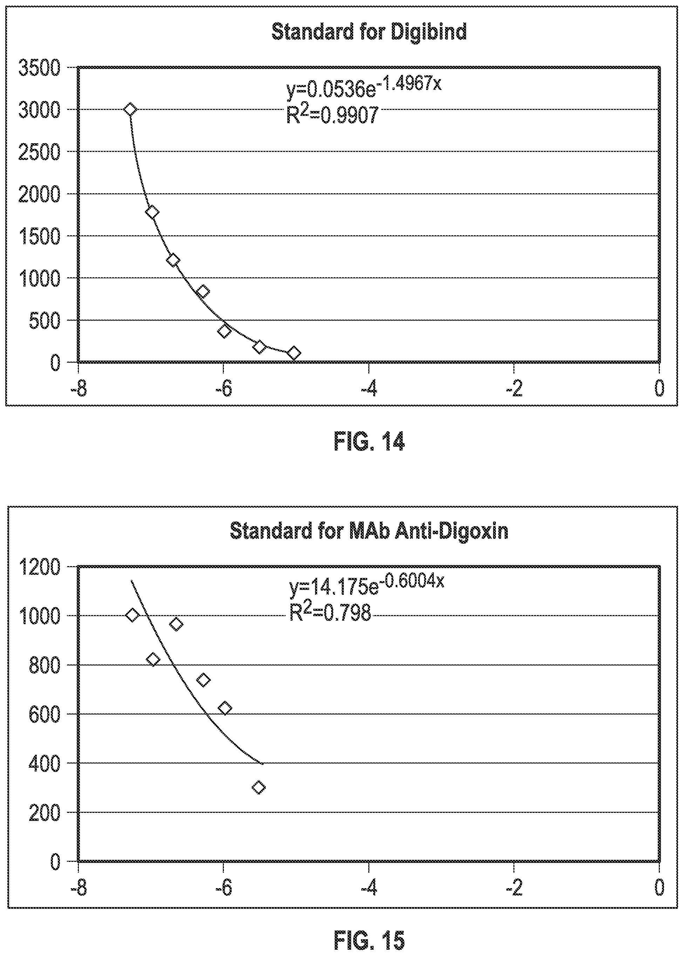

Figure 14 depicts a standard curve for the Digibind RIA. Increasing ouabain concentration (shown as the - log [ouabain]) caused a progressive displacement of [³H]-ouabain from the Digibind. The y-axis displays counts of radioactivity measured after the fixed incubation time as a function of ouabain concentration shown on the x-axis.
Figure 15 depicts a standard Curve for the monoclonal antibody RIA. Increasing ouabain concentration (shown as the - log [ouabain]) caused a progressive displacement of [³H]-ouabain from the Digibind. The y-axis displays counts of radioactivity measured after the fixed incubation time as a function of ouabain concentration shown on the x-axis.

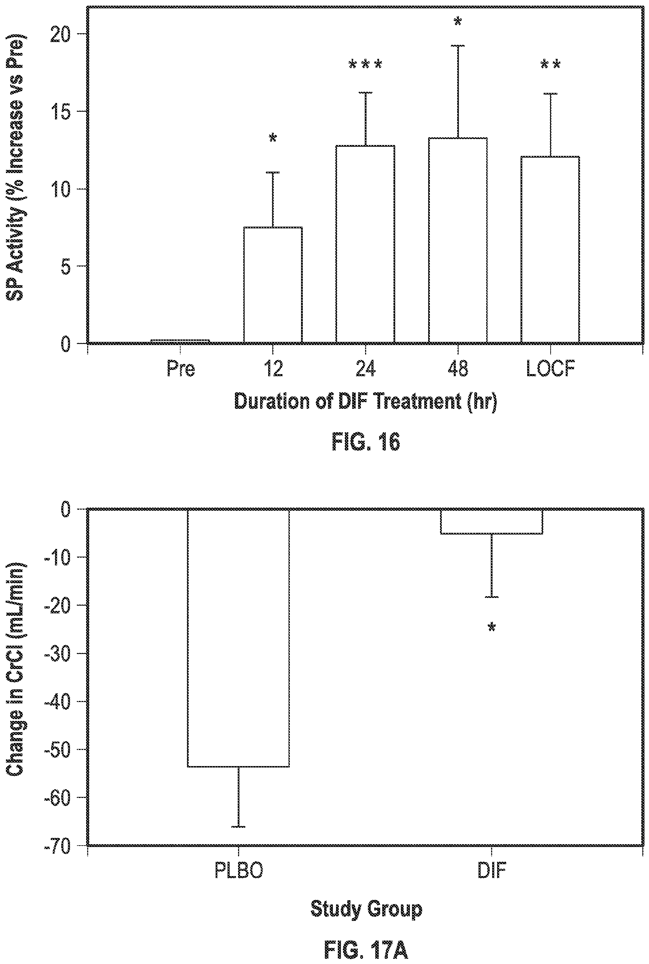

Figure 16 depicts the effect of digoxin immune Fab (DIF) treatment on recovery of sodium pump activity by removal of the endogenous digitalis-like factor (EDLF) in EDLF positive subjects. Relative to the original DEEP study, which included both EDLF positive and negative subjects (15), there was a greater and more significant increase in RBC SP activity/reduction of EDLF in plasma from digoxin immune Fab (DIF) treated women at each time point (t=12 hr: +7.5%, p=0.04; t=24 hr: +12.9%, p=0.0016, t=48 hr: +13.4%, p=0.047, and the last observation carried forward, *i.e.* last observation available: +12.1% increase in SP activity, p=0.010). Subjects receiving placebo showed no change.
Figure 17A depicts the effect of digoxin immune Fab (DIF) treatment versus placebo (PLBO) on the change in CrCl at the 24-48 hours last observation available in EDLF positive subjects (EDLF+ subjects: placebo -53.2 mL/min change in CrCl versus digoxin immune Fab (DIF) -4.5 mL/min change in CrCl, p=0.005).

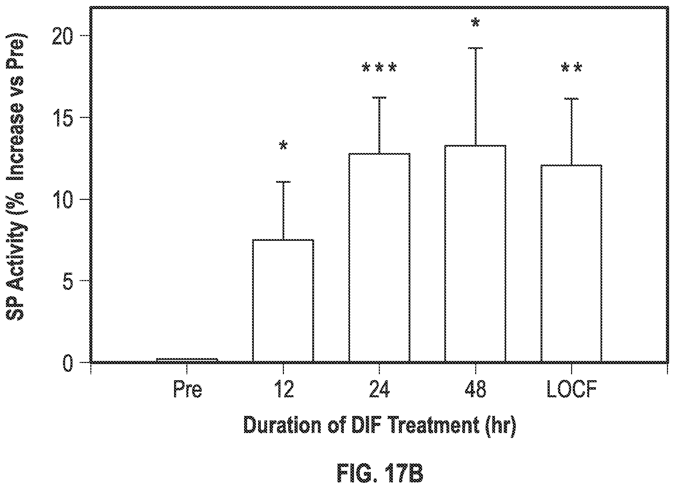

Figure 17B depicts the change in CrCl in PE women receiving placebo according to circulating EDLF levels. Subjects were stratified depending on whether they had no EDLF, low EDLF (1-29 % SP inhibition) or high EDLF (30 % or greater SP inhibition) (p = 0.032).

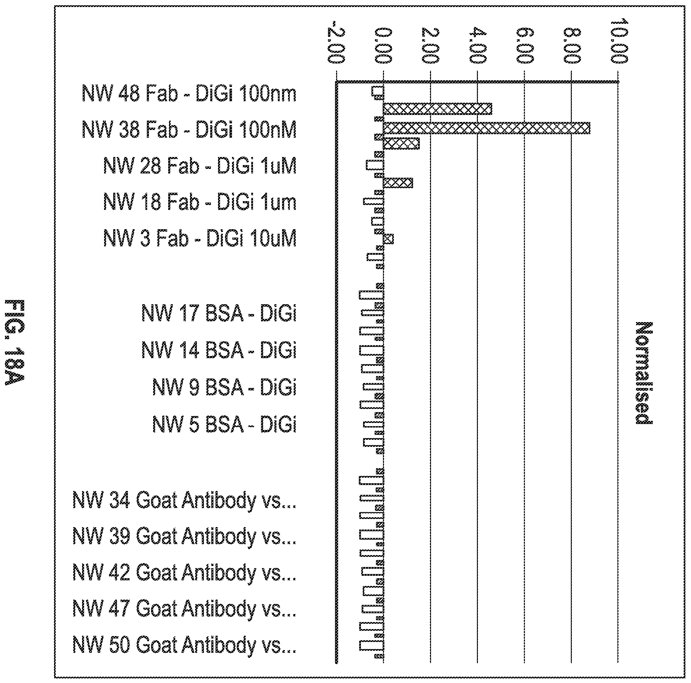

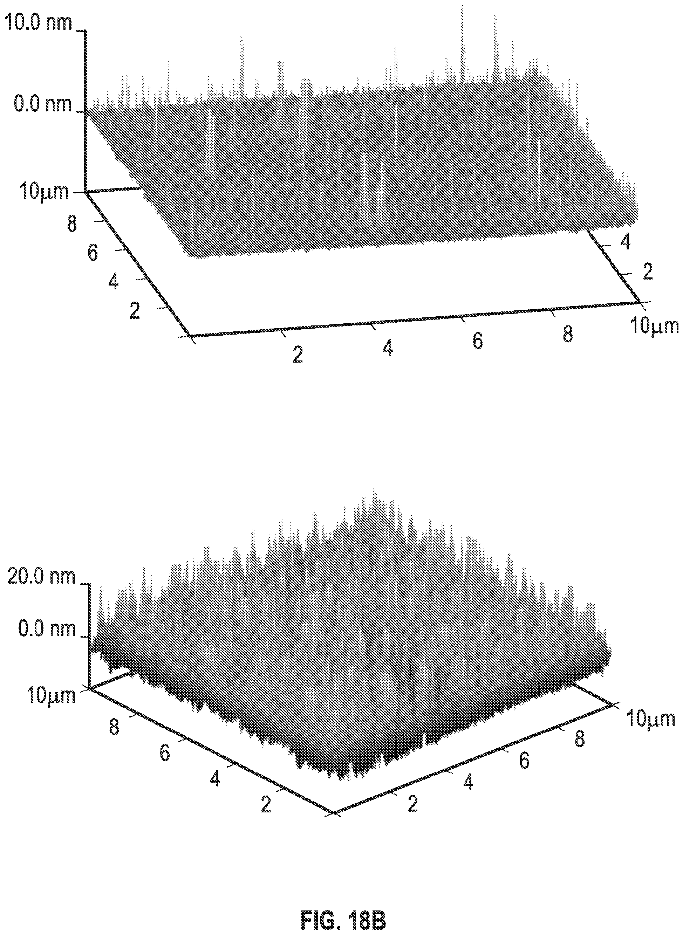

Figure 18A depicts the response of the QMDx NW system to the presence of DiGi on FAB, Goat FAB (control) and BSA (control). The normalised response of the system accounts for starting variability in the NW conductivity profiles. The crosshatched columns indicate positive deviation from the baseline values. Figure 18B depicts atomic force microscopy surface profile of unmodified surface and corresponding FAB modified surface.

### DETAILED DESCRIPTION OF THE PREFERRED EMBODIMENTS

In order that the invention herein described may be fully understood, the following detailed description is set forth. Various embodiments of the invention are described in detail and may be further illustrated by the provided examples. Additional viable variations of the embodiments can easily be envisioned.

### Definitions

Unless defined otherwise, all technical and scientific terms used herein have the same meaning as those commonly understood by one of ordinary skill in the art to which this invention belongs.

As used in the description herein and throughout the claims that follow, the meaning of "a," "an," and "the" includes plural reference unless the context clearly dictates otherwise.

"About," as used herein, will be understood by persons of ordinary skill in the art and will vary to some extent based on the context in which it is used.

"Antibody," as used herein, refers broadly to any polypeptide chain-containing molecular structure with a specific shape that fits to and recognizes an epitope, where one or more non-covalent binding interactions stabilize the complex between the molecular structure and the epitope. The archetypal antibody molecule is the immunoglobulin, and all types of immunoglobulins, IgG, IgM, IgA, IgE, IgD, from all sources, *e.g*., human, rodent, rabbit, cow, sheep, pig, dog, chicken, are considered to be "antibodies." Antibodies include but are not limited to chimeric antibodies, human antibodies and other non-human mammalian antibodies, humanized antibodies, single chain antibodies (scFvs), camelbodies, nanobodies, IgNAR (single-chain antibodies derived from sharks), small-modular immunopharmaceuticals (SMIPs), and antibody fragments (e.g., Fabs, Fab', F(ab')₂.) Numerous antibody coding sequences have been described; and others may be raised by methods well-known in the art. See Streltsov, et al. (2005) Protein Sci. 14(11): 2901-9; Greenberg, et al. (1995) Nature 374(6518): 168-173; Nuttall, et al. (2001) Mol Immunol. 38(4): 313-26; Hamers-Casterman, et al. (1993) Nature 363(6428): 446-8; Gill, et al. (2006) Curr Opin Biotechnol. 17(6): 653-8.

"Diagnostic," as used herein, refers broadly to identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

"Diagnosing," as used herein refers broadly to classifying a disease or a symptom, determining a severity of the disease, monitoring disease progression, forecasting an outcome of a disease and/or prospects of recovery. The term "detecting" may also optionally encompass any of the foregoing. Diagnosis of a disease according to the present invention may, in some embodiments, be affected by determining a level of a polynucleotide or a polypeptide of the present invention in a biological sample obtained from the subject, wherein the level determined can be correlated with predisposition to, or presence or absence of the disease. It should be noted that a "biological sample obtained from the subject" may also optionally comprise a sample that has not been physically removed from the subject.

"Effective amount," as used herein, refers broadly to the amount of a compound, antibody, antigen, or cells that, when administered to a patient for treating a disease, is sufficient to effect such treatment for the disease. The effective amount may be an amount effective for prophylaxis, and/or an amount effective for prevention. The effective amount may be an amount effective to reduce, an amount effective to prevent the incidence of signs/symptoms, to reduce the severity of the incidence of signs/symptoms, to eliminate the incidence of signs/symptoms, to slow the development of the incidence of signs/symptoms, to prevent the development of the incidence of signs/symptoms, and/or effect prophylaxis of the incidence of signs/symptoms. The "effective amount" may vary depending on the disease and its severity and the age, weight, medical history, susceptibility, and pre-existing conditions, of the patient to be treated. The term "effective amount" is synonymous with "therapeutically effective amount" for purposes of this invention.

"Immunoassay," as used herein, refers broadly to an assay that uses an antibody to specifically bind an antigen. The immunoassay may be characterized by the use of specific binding properties of a particular antibody to isolate, target, and/or quantify the antigen.

"Isolated," as used herein, refers broadly to material removed from its original environment in which it naturally occurs, and thus is altered by the hand of man from its natural environment. Isolated material may be, for example, exogenous nucleic acid included in a vector system, exogenous nucleic acid contained within a host cell, or any material which has been removed from its original environment and thus altered by the hand of man (*e.g.,* "isolated antibody").

"Label" or a "detectable moiety" as used herein, refers broadly to a composition detectable by spectroscopic, photochemical, biochemical, immunochemical, chemical, or other physical means.

"Mammal," as used herein, refers broadly to any and all warm-blooded vertebrate animals of the class Mammalia, including humans, characterized by a covering of hair on the skin and, in the female, milk-producing mammary glands for nourishing the young. Examples of mammals include but are not limited to alpacas, armadillos, capybaras, cats, camels, chimpanzees, chinchillas, cattle, dogs, goats, gorillas, hamsters, horses, humans, lemurs, llamas, mice, non-human primates, pigs, rats, sheep, shrews, squirrels, and tapirs. Mammals include but are not limited to bovine, canine, equine, feline, murine, ovine, porcine, primate, and rodent species. Mammal also includes any and all those listed on the Mammal Species of the World maintained by the National Museum of Natural History, Smithsonian Institution in Washington DC.

"Patient," as used herein, refers broadly to any animal who is in need of treatment either to alleviate a disease state or to prevent the occurrence or reoccurrence of a disease state. Also, "Patient" as used herein, refers broadly to any animal who has risk factors, a history of disease, susceptibility, symptoms, signs, was previously diagnosed, is at risk for, or is a member of a patient population for a disease. The patient may be a clinical patient such as a human or a veterinary patient such as a companion, domesticated, livestock, exotic, or zoo animal. The term "subject" may be used interchangeably with the term "patient".

"Subjects" as used herein, refers broadly to anyone suitable to be treated according to the present invention include, but are not limited to, avian and mammalian subjects, and are preferably mammalian. Mammals of the present invention include, but are not limited to, canines, felines, bovines, caprines, equines, ovines, porcines, rodents (*e.g.,* rats and mice), lagomorphs, primates, humans. Any mammalian subject in need of being treated according to the present invention is suitable. Human subjects of both genders and at any stage of development (*i.e.,* neonate, infant, juvenile, adolescent, adult) can be treated according to the present invention. The present invention may also be carried out on animal subjects, particularly mammalian subjects such as mice, rats, dogs, cats, cattle, goats, sheep, and horses for veterinary purposes, and for drug screening and drug development purposes. "Subjects" is used interchangeably with "patients."

"Symptoms" of disease as used herein, refers broadly to any morbid phenomenon or departure from the normal in structure, function, or sensation, experienced by the patient and indicative of disease.

"Therapy," "therapeutic," "treating," or "treatment", as used herein, refers broadly to treating a disease, arresting, or reducing the development of the disease or its clinical symptoms, and/or relieving the disease, causing regression of the disease or its clinical symptoms. Therapy encompasses prophylaxis, treatment, remedy, reduction, alleviation, and/or providing relief from a disease, signs, and/or symptoms of a disease. Therapy encompasses an alleviation of signs and/or symptoms in patients with ongoing disease signs and/or symptoms (*e.g.,* eclampsia, preeclampsia, and/or intraventricular hemorrhage). Therapy also encompasses "prophylaxis". The term "reduced", for purpose of therapy, refers broadly to the clinical significant reduction in signs and/or symptoms. Therapy includes treating relapses or recurrent signs and/or symptoms (*e.g.,* eclampsia, preeclampsia, and/or intraventricular hemorrhage). Therapy encompasses but is not limited to precluding the appearance of signs and/or symptoms anytime as well as reducing existing signs and/or symptoms and eliminating existing signs and/or symptoms. Therapy includes treating chronic disease ("maintenance") and acute disease. For example, treatment includes treating or preventing relapses or the recurrence of signs and/or symptoms (*e.g.,* eclampsia, preeclampsia, and/or intraventricular hemorrhage).

### EDLFS AND ECLAMPSIA/PREECLAMPSIA

Although elevated levels of immunologically detected EDLFs are found both in normal pregnancy and in pregnancy complicated by PE, EDLF levels in PE are substantially higher than in normal pregnancy.

Endogenous digitalis-like factors (EDLFs) appear to be hypertensiogenic and increased in the serum and placenta of women with preeclampsia (PE), a complication of pregnancy. Anti-digoxin antibody Fab fragment (commercially available as Digibind from GlaxoSmithKline and Digifab), reverses in vitro effects of EDLF and in vivo features of PE. Digibind or Digifab in a radioimmunoassay (DigoxinAB RIA) are able to measure EDLF and the quantity of EDLF measured by this assay is comparable to a bio-functional assay of EDLF. The human placenta was a source of EDLF, synthesizing and releasing EDLF into the media of cultured human placental tissue. Ketoconazole, a steroid synthesis inhibitor, and 17-OH progesterone were shown to inhibit or increase EDLF release, respectively, evidencing overlap of synthetic pathways. Abnormalities of PE such as placental hypoxia, increased reactive oxygen species and increased pro-inflammatory cytokines were demonstrated to increase placental EDLF release. Regulated secretion of EDLFs in response to factors thought to mediate PE represents a marked advance in this research. Hypoxia, oxidative stress, and cytokines have all been shown to be increased in PE and the present inventors show that these factors that will modulate EDLF production. The inventors surprisingly discovered that ketoconazole and 17α-hydroxyprogesterone alter EDLF production and thereby may be used to regulate the EDLF synthetic pathway. Thus DigoxinAB may improve the symptoms of PE, especially hypertension and it could be used in a therapy for PE. This has been recently tested in a clinical trial of Digibind in women with severe PE with positive results. Adair, et al. (2010) Amer J Perinatol. 27: 655-662.

### Determine the Synthetic Pathway and Regulation of EDLF in Human Placenta.

Historically, placenta have been considered a necessary participant in the development of preeclampsia (PE). Removal of the placenta/fetus brings about a rapid resolution of all features of PE.

EDLFs may mediate several features of PE, but the placenta has not been seriously considered as a source for EDLFs. Recent research has documented exceptionally high levels of EDLF in placenta, especially from preeclamptic pregnancies. Hopoate-Sitake, et al. (2011) Reproductive Sci 18: 190-199. The placenta can synthesize and release EDLFs. Placental tissue has been shown to have high levels of one or more EDLFs. Hopoate-Sitake, et al. (2011) Reproductive Sci 18: 190-199. Placental tissue produces and releases EDLFs. Ketoconazole, a steroid synthesis blocker, markedly reduced placental EDLF production in a dose-dependent manner, whereas, 17-OH progesterone, which can act as a substrate for steroid synthesis increased synthesis and release of EDLF. Progesterone itself appeared to have little or no effect on EDLF production. The difference in the placental response to these two steroids may have to do with the absence of a 17-hydroxylase in placenta which would limit the placenta's ability to process progesterone further to products that require a 17-OH group, even as an intermediate. See Ugele, et al. (1999) J Steroid Biochem Mol Biol. 71: 203-211. EDLFs are produced in the placenta and involve enzymes involved in steroid synthesis.

Several circulating or locally acting regulatory compounds that have been demonstrated to be present at abnormal levels in PE pregnancies. Some of these are higher levels of pro-inflammatory cytokines, low oxygen, increased reactive oxygen species and increased pro-angiogenic/decreased anti-angiogenic factors. Tumor necrosis factor-α (a pro-inflammatory cytokine) caused marked increases in EDLF release from normal human placenta as did low concentration of H2O2 a reactive oxygen species. Low oxygen appeared to increase EDLF output modestly.

Early studies have been carried out experiments with interleukin-6 (a second pro-inflammatory cytokine), pregnenolone (an early steroid pathway intermediate), placental growth factor, vascular endothelial growth factor (both pro-angiogenic factors) and sFLT-1 (a soluble receptor that is anti-angiogenic). All of these are increased in PE, and explain the reason for EDLFs becoming increased in PE.

### Isolate EDLF from Human Placenta and Characterize its Chemical Structure.

Endogenous digitalis-like factors (EDLFs) are compounds produced in the body which circulate in the blood. EDLFs participate in hypertensive disease and in kidney function. These compounds as having a direct role in the most common form of high blood pressure. Haddy, et al. (1995) "Endogenous digitalis-like factors in hypertension." In Brenner and Laragh (Eds) Hypertension: Pathophysiology, Diagnosis, and Management. Raven Press, New York, pages 1055-1067; Graves, et al. (1987) Ann Rev Med 38: 433-444. EDLFs play an active role in preeclampsia (PE) and studies to date suggest that EDLFs may mediate high blood pressure in many PE women. Graves, et al. (1984) J Clin Endocrinol Metab 59: 1070-4; Graves (1987) Hypertension 10(S Pt 2): I-84-6; Graves (2007) Frontiers in Bioscience 12: 2438-2446; Goodlin (1988) N Eng J Med 318: 518-519; Adair, et al. (1997) Am J Hypertens 10: 11A; Adair, et al. (1996) Am J Nephrol 16: 529-531; and Adair, et al. (2009) J Perinatol 29: 284-9.

EDLFs was isolated from human placental specimens by affinity chromatography using Digibind as the antibody binding agent. This antibody affinity isolation of EDLFs coupled with ultrafiltration techniques allows for purification of EDLF from biological specimens in a relatively short time. The inventors surprisingly discovered that human placenta, even from uncomplicated pregnancies, has abundant EDLF. After the antibody separation of EDLF from homogenates, the isolated EDLFs can then be further separated and purified by HPLC chromatography techniques leading to highly purified material. The EDLF structure was analyzed by various chemical analyses, and mass spectrometry (MS).

Ouabain, may represent one EDLF or at least be chemically analogous to EDLF. Ouabain was successfully ionized and analyzed by MS providing a characteristic elution time and mass spectrum. In addition, successful fragmentation of the intact molecule was accomplished using tandem MS-MS techniques confirming that structural information can be obtained.

The high levels of an EDLF in placental homogenates were found. Placental production of EDLF was increased by hydrogen peroxide or tumor necrosis factor α.

Using Digibind to selectively bind the EDLF, substantial quantities (*e.g., ∼*80%) of the inhibitor were removed from tissue homogenates. Using ultrafiltration and agents to release the EDLF at certain stages in the purification eliminated most unwanted impurities. HPLC separation methods then was used to further isolate and purify one or more EDLFs. Using the EDLF assay it was determined where the EDLFs eluted from in the separation column. The individual EDLFs can then be collected, stockpiled and submitted to mass spectrometry analysis. Capillary liquid chromatography may be coupled to one of several mass spectrometers.

### Antibodies

Antibodies may comprise of two identical light polypeptide chains of molecular weight approximately 23,000 daltons ("light chain"), and two identical heavy chains of molecular weight 53,000-70,000 ("heavy chain"). See Edelman (1971) Ann. NY. Acad. Sci. 190: 5. The four chains are joined by disulfide bonds in a "Y" configuration wherein the light chains bracket the heavy chains starting at the mouth of the "Y" configuration. The "branch" portion of the "Y" configuration is designated the Fab region; the stem portion of the "Y" configuration is designated the Fc region. The amino acid sequence orientation runs from the N-terminal end at the top of the "Y" configuration to the C-terminal end at the bottom of each chain. The N-terminal end possesses the variable region having specificity for the antigen that elicited it, and is about 100 amino acids in length, there being slight variations between light and heavy chain and from antibody to antibody.

The variable region is linked in each chain to a constant region that extends the remaining length of the chain and that within a particular class of antibody does not vary with the specificity of the antibody (*i.e.,* the antigen eliciting it). There are five known major classes of constant regions that determine the class of the immunoglobulin molecule (*e.g.,* IgG, IgM, IgA, IgD, and IgE corresponding to γ, µ, α, δ, and ε heavy chain constant regions). The antibodies and antibody fragments decribed herein may be human, humanized, murine, ovine, bovine, or porcine. The antibody fragments described herein may be an Fab fragment.

The constant region or class determines subsequent effector function of the antibody, including activation of complement (Kabat (1976) Structural Concepts in Immunology and Immunochemistry [2nd Ed.] pages 413-436; Holt, Rinehart, Winston) and other cellular responses (Andrews, et al. (1980) Clinical Immunobiology 1-18; Kohl, et al. (1983) Immunology 48: 187) while the variable region determines the antigen with which it will react. Light chains are classified as either κ (kappa) or λ (lambda). Each heavy chain class may be prepared with either kappa or lambda light chain. The light and heavy chains are covalently bonded to each other, and the "tail" portions of the two heavy chains are bonded to each other by covalent disulfide linkages when the immunoglobulins are generated either by hybridomas or by B cells.

Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein. *See, e.g.,* Harlow & Lane (1998) USING ANTIBODIES: A LABORATORY MANUAL Cold Spring Harbor Laboratory, for a description of immunoassay formats and conditions that can be used to determine specific immunoreactivity. Typically a specific or selective reaction will be at least twice background signal or noise and more typically more than about 10 to 100 times background.

### Antibody Fragments

In addition to entire immunoglobulins (or their recombinant counterparts), immunoglobulin fragments comprising the epitope binding site (*e.g.,* Fab', F(ab')₂, or other fragments) may be synthesized. "Fragment," or minimal immunoglobulins may be designed utilizing recombinant immunoglobulin techniques. For instance "Fv" immunoglobulins for use in the present invention may be produced by synthesizing a fused variable light chain region and a variable heavy chain region. Combinations of antibodies are also of interest, *e.g.* diabodies, which comprise two distinct Fv specificities. Antigen-binding fragments of immunoglobulins include but are not limited to SMIPs (small molecule immunopharmaceuticals), camelbodies, nanobodies, and IgNAR.

The anti-digoxin Fab (DIF) may be an Fab antibody fragment that selectively binds to a digoxindicarboxymethoxylamine (DDMA), a digoxin derivative. The anti-digoxin Fab (DIF) may be Digoxin Immune Fab, Digibind, DigiFab, or combinations thereof. Anti-digoxin antibodies may be antibodies that selectively bind digoxin. Antman, et al. Circulation 1990; 81:1744; Sinclair, et al. Br J Clin Pharmacol. 1989, 28(3): 352-356.

Anti-EDLF antibodies and antibody fragments thereof may be used in the methods described herein. Anti-EDLF antibodies and fragments thereof are described in, for instance, WO 1994/012210.

### PHARMACEUTICAL COMPOSITIONS

A "pharmaceutical composition" refers to a chemical or biological composition suitable for administration to a mammal. Such compositions may be specifically formulated for administration via one or more of a number of routes, including but not limited to buccal, epicutaneous, epidural, inhalation, intraarterial, intracardial, intracerebroventricular, intradermal, intramuscular, intranasal, intraocular, intraperitoneal, intraspinal, intrathecal, intravenous, oral, parenteral, rectally via an enema or suppository, subcutaneous, subdermal, sublingual, transdermal, and transmucosal. In addition, administration may occur by means of injection, powder, liquid, gel, drops, or other means of administration.

A "pharmaceutical excipient" or a "pharmaceutically acceptable excipient" is a carrier, usually a liquid, in which an active therapeutic agent is formulated. In one embodiment of the invention, the active therapeutic agent is a humanized antibody described herein, or one or more fragments thereof. The excipient generally does not provide any pharmacological activity to the formulation, though it may provide chemical and/or biological stability, and release characteristics. Exemplary formulations may be found, for example, in Grennaro (2005) [Ed.] Remington: The Science and Practice of Pharmacy [21st Ed.]

Pharmaceutical compositions typically must be sterile and stable under the conditions of manufacture and storage. The invention contemplates that the pharmaceutical composition is present in lyophilized form. The composition may be formulated as a solution, microemulsion, liposome, or other ordered structure suitable to high drug concentration. The carrier may be a solvent or dispersion medium containing, for example, water, ethanol, polyol (for example, glycerol, propylene glycol, and liquid polyethylene glycol), and suitable mixtures thereof. The invention further contemplates the inclusion of a stabilizer in the pharmaceutical composition.

The anti-digoxin antibodies and fragments thereof, of the present invention thereof may be formulated into pharmaceutical compositions of various dosage forms. To prepare the pharmaceutical compositions of the invention, at least one anti-digoxin antibody, or binding fragments thereof, as the active ingredient may be intimately mixed with appropriate carriers and additives according to techniques well known to those skilled in the art of pharmaceutical formulations. See Grennaro (2005) [Ed.] Remington: The Science and Practice of Pharmacy [21st Ed.] For example, the antibodies described herein may be formulated in phosphate buffered saline pH 7.2 and supplied as a 5.0 mg/mL clear colorless liquid solution.

Similarly, compositions for liquid preparations include solutions, emulsions, dispersions, suspensions, syrups, and elixirs, with suitable carriers and additives including but not limited to water, alcohols, oils, glycols, preservatives, flavoring agents, coloring agents, and suspending agents. Typical preparations for parenteral administration comprise the active ingredient with a carrier such as sterile water or parenterally acceptable oil including but not limited to polyethylene glycol, polyvinyl pyrrolidone, lecithin, arachis oil or sesame oil, with other additives for aiding solubility or preservation may also be included. In the case of a solution, it may be lyophilized to a powder and then reconstituted immediately prior to use. For dispersions and suspensions, appropriate carriers and additives include aqueous gums, celluloses, silicates, or oils.

For each of the recited embodiments, the anti-digoxin antibody, or antigen-binding fragments thereof, may be administered by a variety of dosage forms. Any biologically-acceptable dosage form known to persons of ordinary skill in the art, and combinations thereof, are contemplated. Examples of such dosage forms include, without limitation, reconstitutable powders, elixirs, liquids, solutions, suspensions, emulsions, powders, granules, particles, microparticles, dispersible granules, cachets, inhalants, aerosol inhalants, patches, particle inhalants, implants, depot implants, injectables (including subcutaneous, intramuscular, intravenous, and intradermal), infusions, and combinations thereof.

In many cases, it will be preferable to include isotonic agents, *e.g*., sugars, polyalcohols such as mannitol, sorbitol, or sodium chloride in the composition. Prolonged absorption of the injectable compositions may be brought about by including in the composition an agent which delays absorption, *e.g*., monostearate salts and gelatin. Moreover, the compositions described herein may be formulated in a time release formulation, *e.g.* in a composition that includes a slow release polymer. The anti-digoxin antibody, or antigen-binding fragments thereof, may be prepared with carriers that will protect the compound against rapid release, such as a controlled release formulation, including implants and microencapsulated delivery systems. Biodegradable, biocompatible polymers may be used, such as ethylene vinyl acetate, polyanhydrides, polyglycolic acid, collagen, polyorthoesters, polylactic acid and polylactic, polyglycolic copolymers (PLG). Many methods for the preparation of such formulations are known to those skilled in the art.

A person of skill in the art would be able to determine an effective dosage and frequency of administration through routine experimentation, for example guided by the disclosure herein and the teachings in Goodman, et al. (2011) Goodman & Gilman's The Pharmacological Basis of Therapeutics [12th Ed.]; Howland, et al. (2005) Lippincott's Illustrated Reviews: Pharmacology [2nd Ed.]; and Golan, (2008) Principles of Pharmacology: The Pathophysiologic Basis of Drug Therapy [2nd Ed.] See, also, Grennaro (2005) [Ed.] Remington: The Science and Practice of Pharmacy [21st Ed.]

### Routes of Administration

The compositions described herein may be administered in any of the following routes: buccal, epicutaneous, epidural, infusion, inhalation, intraarterial, intracardial, intracerebroventricular, intradermal, intramuscular, intranasal, intraocular, intraperitoneal, intraspinal, intrathecal, intravenous, oral, parenteral, pulmonary, rectally via an enema or suppository, subcutaneous, subdermal, sublingual, transdermal, and transmucosal. The preferred routes of administration are intravenous injection or infusion. The administration can be local, where the composition is administered directly, close to, in the locality, near, at, about, or in the vicinity of, the site(s) of disease, wherein the composition is given to the patient and passes through the body widely, thereby reaching the site(s) of disease. Local administration (*e.g.,* injection) may be accomplished by administration to the cell, tissue, organ, and/or organ system, which encompasses and/or is affected by the disease, and/or where the disease signs and/or symptoms are active or are likely to occur (*e.g.,* placenta). Administration can be topical with a local effect, composition is applied directly where its action is desired (*e.g.,* placenta).

For each of the recited embodiments, the anti-digoxin antibodies or antigen-binding fragments can be administered by a variety of dosage forms as known in the art. Any biologically-acceptable dosage form known to persons of ordinary skill in the art, and combinations thereof, are contemplated. Examples of such dosage forms include, without limitation, chewable tablets, quick dissolve tablets, effervescent tablets, reconstitutable powders, elixirs, liquids, solutions, suspensions, emulsions, tablets, multilayer tablets, bi-layer tablets, capsules, soft gelatin capsules, hard gelatin capsules, caplets, lozenges, chewable lozenges, beads, powders, gum, granules, particles, microparticles, dispersible granules, cachets, douches, suppositories, creams, topicals, inhalants, aerosol inhalants, patches, particle inhalants, implants, depot implants, ingestibles, injectables (including subcutaneous, intramuscular, intravenous, and intradermal), infusions, and combinations thereof.

Other compounds which can be included by admixture are, for example, medically inert ingredients (*e.g.,* solid and liquid diluent), such as lactose, dextrosesaccharose, cellulose, starch or calcium phosphate for tablets or capsules, olive oil or ethyl oleate for soft capsules and water or vegetable oil for suspensions or emulsions; lubricating agents such as silica, talc, stearic acid, magnesium or calcium stearate and/or polyethylene glycols; gelling agents such as colloidal clays; thickening agents such as gum tragacanth or sodium alginate, binding agents such as starches, arabic gums, gelatin, methylcellulose, carboxymethylcellulose or polyvinylpyrrolidone; disintegrating agents such as starch, alginic acid, alginates or sodium starch glycolate; effervescing mixtures; dyestuff; sweeteners; wetting agents such as lecithin, polysorbates or laurylsulphates; and other therapeutically acceptable accessory ingredients, such as humectants, preservatives, buffers and antioxidants, which are known additives for such formulations.

Liquid dispersions for oral administration can be syrups, emulsions, solutions, or suspensions. The syrups can contain as a carrier, for example, saccharose or saccharose with glycerol and/or mannitol and/or sorbitol. The suspensions and the emulsions can contain a carrier, for example a natural gum, agar, sodium alginate, pectin, methylcellulose, carboxymethylcellulose, or polyvinyl alcohol.

In further embodiments, the present invention provides kits including one or more containers comprising pharmaceutical dosage units comprising an effective amount of anti-digoxin antibodies and antibody fragments thereof of the present invention. Kits may include instructions, directions, labels, marketing information, warnings, or information pamphlets.

### Dosages

The amount of EDLF, antibodies and antigen-binding fragments thereof, in a therapeutic composition according to any embodiments of this invention may vary according to factors such as the disease state, age, gender, weight, patient history, risk factors, predisposition to disease, administration route, pre-existing treatment regime (*e.g.,* possible interactions with other medications), and weight of the individual. Dosage regimens may be adjusted to provide the optimum therapeutic response. For example, a single bolus may be administered, several divided doses may be administered over time, or the dose may be proportionally reduced or increased as indicated by the exigencies of therapeutic situation.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of antibodies, and fragments thereof, calculated to produce the desired therapeutic effect in association with the required pharmaceutical carrier. The specification for the dosage unit forms of the invention are dictated by and directly dependent on the unique characteristics of the antibodies, and fragments thereof, and the particular therapeutic effect to be achieved, and the limitations inherent in the art of compounding such an antibodies, and fragments thereof, for the treatment of sensitivity in individuals. In therapeutic use for treatment of conditions in mammals (*e.g.,* humans) for which the antibodies and fragments thereof of the present invention or an appropriate pharmaceutical composition thereof are effective, the antibodies and fragments thereof of the present invention may be administered in an effective amount. The dosages as suitable for this invention may be a composition, a pharmaceutical composition or any other compositions described herein.

The dosage may be administered as a single dose, a double dose, a triple dose, a quadruple dose, and/or a quintuple dose. The dosages may be administered singularly, simultaneously, and sequentially.

The dosage form may be any form of release known to persons of ordinary skill in the art. The compositions of the present invention may be formulated to provide immediate release of the active ingredient or sustained or controlled release of the active ingredient. In a sustained release or controlled release preparation, release of the active ingredient may occur at a rate such that blood levels are maintained within a therapeutic range but below toxic levels over an extended period of time (*e.g.,* 4 to 24 hours). The preferred dosage forms include immediate release, extended release, pulse release, variable release, controlled release, timed release, sustained release, delayed release, long acting, and combinations thereof, and are known in the art.

It will be appreciated that the pharmacological activity of the compositions may be monitored using standard pharmacological models that are known in the art. Furthermore, it will be appreciated that the compositions comprising a EDLFs, antibody or antigen-binding fragment thereof, may be incorporated or encapsulated in a suitable polymer matrix or membrane for site-specific delivery, or may be functionalized with specific targeting agents capable of effecting site specific delivery. These techniques, as well as other drug delivery techniques are well known in the art. Determination of optimal dosages for a particular situation is within the capabilities of those skilled in the art. See, *e.g.,* Grennaro (2005) [Ed.] Remington: The Science and Practice of Pharmacy [21st Ed.]

### METHODS OF TREATMENT

The invention provides for the treatment of eclampsia or preeclampsia comprising determining if a patient with eclampsia or preeclampsia or at risk for eclampsia or preeclampsia has elevated levels of EDLF, and if ELDF positive, administering an anti-digoxin antibody or antibody fragment thereof, optionally an Fab fragment.

Also, a gravid human patient exhibiting at least one symptom of gestational hypertension, preeclampsia, eclampsia, or intrauterine growth restriction may be tested for EDLF levels and, if ELDF positive, administering an anti-digoxin antibody or antibody fragment thereof, optionally an Fab fragment

A gravid human patient who exhibits at least one symptom of gestational hypertension, preeclampsia, eclampsia, or intrauterine growth restriction may be tested for EDLF by obtaining a sample, optionally a blood, serum, or placenta sample, and assaying the same with a digoxin-immune antibody immunoassay to determine whether the patient is EDLF positive based on immunoassay, and administering anti-digoxin antibody or antibody fragment thereof, optionally an Fab fragment to patient if the patient is determined to be EDLF positive.

Also, intraventricular hemorrhage (IVH) in the neonate of the patient may be prevented by conducting a digoxin-immune antibody immunoassay on the patient to determine whether the patient is EDLF positive based on immunoassay; and administering anti-digoxin antibody or antibody fragment thereof, optionally an Fab fragment to patient if the patient is determined to be EDLF positive.

Intraventricular hemorrhage may be treated in a patient in need thereof comprising conducting a digoxin-immune antibody immunoassay on a gravid human patient whose fetus may develop IVH as a result of being delivered prematurely (before 40 weeks gestation) to determine whether the patient is EDLF positive based on immunoassay, and administering anti-digoxin antibody or antibody fragment thereof, optionally an Fab fragment to patient if the patient is determined to be EDLF positive.

An anti-digoxin antibody or antigen-binding fragment thereof may be administered to prevent intraventricular hemorrhage (IVH) in the neonate of the patient comprising conducting a digoxin-immune antibody immunoassay on a gravid human patient whose fetus may develop IVH as a result of being delivered prematurely (before 40 weeks gestation) to determine whether the patient is EDLF positive based on radioimmunoassay; and administering anti-digoxin antibody or antibody fragment thereof, optionally an Fab fragment to patient if the patient is determined to be EDLF positive.

Methods for treating fetal complications associated with premature birth, including may comprise conducting a digoxin-immune antibody immunoassay on a gravid human patient whose fetus may be delivered prematurely (before 40 weeks gestation) to determine whether the patient is EDLF positive based on immunoassay; and administering anti-digoxin antibody or antibody fragment thereof, optionally an Fab fragment to patient if the patient is determined to be EDLF positive.

The fetal complications associated with premature birth may include IVH or NEC.

The method of extending pregnancy in a gravid human patient exhibiting at least one symptom of gestational hypertension, preeclampsia, eclampsia, or intrauterine growth restriction may comprise: (a) conducting a digoxin-immune antibody radioimmunoassay on a patient suffering from eclampsia or preeclampsia; (b) determining whether the patient is EDLF positive based on radioimmunoassay; and (c) administering digoxin immune Fab (DIF) to patient if the patient is determined to be EDLF positive.

The method for treating a patient at risk for eclampsia or preeclampsia may comprise: (a) obtaining a sample from a patient at risk for eclampsia or preeclampsia; (b) contacting said sample with an anti-EDLF antibody or antibody fragment thereof; (c) detecting the presence of an anti-EDLF antibody or antibody fragment-EDLF complex, wherein the presence of said EDLF is indicative of eclampsia or preeclampsia; and (d) administering digoxin immune Fab (DIF) to patient if the patient is determined to be EDLF positive.

The method for treating a patient whose fetus and/or neonate is at risk for IVH may comprise: (a) obtaining a sample from a patient; (b) contacting said sample with an anti-EDLF antibody or antibody fragment thereof; (c) detecting the presence of an anti-EDLF antibody or antibody fragment-EDLF complex, wherein the presence of said EDLF is indicative of eclampsia or preeclampsia; and (d) administering digoxin immune Fab (DIF) to patient if the patient is determined to be EDLF positive.

The method for screening patients for responsiveness to anti-digoxin therapy for eclampsia or preeclampsia:(a) obtaining a sample from a patient at risk for eclampsia or preeclampsia; (b) assaying for the presence of EDLF; (c) determining the EDLF level; (d) administering an anti-digoxin antibody or antibody fragment thereof to said patient if said EDLF level is over 100 nm EDLF.

The method for screening patients for responsiveness to anti-digoxin therapy for gestational hypertension, preeclampsia, eclampsia, or intrauterine growth restriction may comprise:(a) obtaining a sample from a patient suffering from gestational hypertension, preeclampsia, eclampsia, or intrauterine growth restriction; (b) assaying for the presence of EDLF; (c) administering an anti-digoxin antibody or antibody fragment thereof to said patient if the patient is determined to be EDLF positive.

The method for screening patients for responsiveness to anti-digoxin therapy for gestational hypertension, preeclampsia, eclampsia, or intrauterine growth restriction may comprise: (a) obtaining a sample from a patient at risk for gestational hypertension, preeclampsia, eclampsia, or intrauterine growth restriction; (b) assaying for the presence of EDLF; (c) administering an anti-digoxin antibody or antibody fragment thereof to said patient if the patient is determined to be EDLF positive.

The method for screening patients for responsiveness to anti-digoxin therapy for gestational hypertension, preeclampsia, eclampsia, or intrauterine growth restriction may comprise:(a) obtaining a sample from a patient at risk for gestational hypertension, preeclampsia, eclampsia, or intrauterine growth restriction; (b) assaying for the presence of EDLF;(c) determining the EDLF level; (d) administering an anti-digoxin antibody or antibody fragment thereof to said patient if said EDLF level is over 100 nm EDLF.

The method of administering anti-digoxin antibody or antigen-binding fragment thereof to treat intraventricular hemorrhage may comprise: (a) conducting a digoxin-immune antibody radioimmunoassay on a patient suffering from intraventricular hemorrhage; (b) determining whether the patient is EDLF positive based on radioimmunoassay; and (c) administering the anti-digoxin antibody or antigen-binding fragment thereof to patient if the patient is determined to be EDLF positive.

The method for treating intraventricular hemorrhage may comprise: (a) obtaining a sample from a patient whose fetus is at risk for intraventricular hemorrhage; (b) assaying for the presence of EDLF; (c) determining the EDLF level; (d) administering an anti-digoxin antibody or antibody fragment thereof to said patient if said EDLF level is over 100 nm EDLF.

The method for screening patients for responsiveness to anti-digoxin therapy for intraventricular hemorrhage: (a) obtaining a sample from a patient at risk for intraventricular hemorrhage; (b) assaying for the presence of EDLF; (c) determining the EDLF level; (d) administering an anti-digoxin antibody or antibody fragment thereof to said patient if said EDLF level is over 100 nm EDLF.

The method for treating a patient at risk for intraventricular hemorrhage may comprise: (a) obtaining a sample from a patient at risk for intraventricular hemorrhage; (b) contacting said sample with an anti-EDLF antibody or antibody fragment thereof; and (c) detecting the presence of an anti-EDLF antibody or antibody fragment-EDLF complex, wherein the presence of said EDLF is indicative of intraventricular hemorrhage.

The antibody-fragment may be a Fab, Fab', F(ab')2, Fv, CDR, paratope, or portion of an antibody that is capable of binding the antigen.

The antibody may be chimeric, humanized, anti-idiotypic, single-chain, bifunctional, or co-specific.

The antibody or fragment may be conjugated to a label. In another embodiment, label may be a chemiluminescent label, paramagnetic label, an MRI contrast agent, fluorescent label, bioluminescent label, or radioactive label. In another embodiment, paramagnetic label may be aluminum, manganese, platinum, oxygen, lanthanum, lutetium, scandium, yttrium, or gallium.

The antibody may be attached to a solid support. The solid phase support may be a bead, test tube, sheet, culture dish, nanowire or test strip. The solid support may be an array. The nanowire may be in array. The Hemmilä, et al. "Integration of microfluidic sample delivery system on silicon nanowire-based biosensor." Microsyste. Techol., 2014. The anti-digoxin antibody may be immobilized on a nanowire biosensor.

A silicon nanowire biosensor may comprise an immobilized anti-digoxin antibody, optionally an anti-digoxin Fab antibody fragment. The fragment may be a Fab, Fab', F(ab')2, Fv, CDR, paratope, or portion of an antibody that is capable of binding the antigen. The antibody may be chimeric, humanized, anti-idiotypic, single-chain, bifunctional, or co-specific. The biosensor may have a sensativity of at least about 100 nM of digoxin in a biological sample.

The method of detecting EDLF may comprise contacting a biological sample with a nanowire biosensor comprising an immobilized anti-digoxin antibody, optionally an anti-digoxin Fab antibody fragment, and assaying for the presence of EDLF.

A method for screening patients for responsiveness to anti-digoxin therapy for eclampsia or preeclampsia may comprise (a) obtaining a sample from a patient at risk for eclampsia or preeclampsia, optionally a blood sample; (b) assaying for the presence of EDLF comprising contacting said biological sample with a nanowire biosensor; (c) determining the EDLF level, wherein an EDLF level above about 100 nM is indicative of responsiveness to anti-digoxin therapy for eclampsia or preeclampsia.

A method for screening patients for responsiveness to anti-digoxin therapy for gestational hypertension, preeclampsia, eclampsia, or intrauterine growth restriction may comprise (a) obtaining a sample from a patient at risk for gestational hypertension, preeclampsia, eclampsia, or intrauterine growth restriction, optionally a blood sample; (b) assaying for the presence of EDLF comprising contacting said biological sample with a nanowire biosensor; (c) determining the EDLF level; wherein an EDLF level over 100 nM is indicative of responsiveness to anti-digoxin therapy for gestational hypertension, preeclampsia, eclampsia, or intrauterine growth restriction.

A method for screening patients for eclampsia or preeclampsia may comprise (a) obtaining a sample from a patient at risk for eclampsia or preeclampsia, optionally a blood sample; (b) assaying for the presence of EDLF comprising contacting said biological sample with a nanowire biosensor; (c) determining the EDLF level; wherein an EDLF level above 100 nM is indicative of eclampsia or preeclampsia.

The sample may be a blood, serum, plasma, or placenta sample.

The antibody immunoassay may be an assay selected from the group consisting of Western blots, radioimmunoassays, ELISA (enzyme linked immunosorbent assay), "sandwich" immunoassays, immunoprecipitation assays, precipitation reactions, gel diffusion precipitation reactions, immunodiffusion assays, agglutination assays, complement-fixation assays, immunohistochemical assays, fluorescent immunoassays, and protein A immunoassays.

The EDLF level may be over 100 nM EDLF. The EDLF level may be about 100, 200, 300, 400, or 500 nM EDLF. The EDLF level may be over about 100, 200, 300, 400, or 500 nM EDLF.

The administered dosage of digoxin antibody may be at least than 0.006 mg digoxin binding capacity /Kg. The administered dosage of digoxin antibody may be at least than 0.001, 0.002, 0.003, 0.004, 0.005, 0.006, 0.007, 0.008, 0.009, or 0.010 mg digoxin binding capacity /Kg. The dosage may be administered over a period of six hours or less. The dosage may be administered over a period of about 1, 2, 3, 4, 5, or 6 hours.

The antibody fragments may be administered intravenously in 0.9% (w/v) sodium chloride, or deionized water. The anti-digoxin antibody or anti-body fragment thereof may be humanized or chimeric.

The methods of treatment described herein may further comprise administration of subsequent dosages of digoxin immune Fab. The methods of treatment described herein may further comprise administering a therapeutically effective amount of corticosteroid. The methods of treatment described herein may further comprise administration of subsequent dosages of digoxin immune Fab.

The methods of treatment described herein may further comprise administering a therapeutically effective amount of an antihypertensive drug. The antihypertensive drug may be labetalol, altenolol, nifedipine, 1-methyldopa or hydralazine.

The methods of treatment described herein may further comprise administering a therapeutically effective amount of magnesium sulfate or phenytoin. The digoxin immune Fab may be ovine digoxin immune Fab. The dose may be no more than approximately 10.0 mg. The dose may be no more than approximately 5.0 mg. The dose may be in the range between approximately 0.01 to 1.0 mg. The dose may be in the range between approximately 0.01 mg to 0.5 mg. The dose may be in about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, or 10 mg.

### DIAGNOSTIC METHODS

Anti-EDLF and antibody-fragments thereof may be used in diagnostic methods for detecting the presence or absence of EDLF. The anti-EDLF antibody and antigen-binding fragments thereof, may be used in methods comprising (a) contacting a test sample with an antibody, or fragment thereof, that binds a EDLF, and (b) assaying for antibody-epitope complexes, wherein the presence of said epitope is indicative of a carcinoma. Further, the Anti-EDLF antibodies, may be used in a method for detecting the presence of a EDLF in a patient comprising (a) administering to said patient a labeled monoclonal antibody, or fragment thereof, that binds a EDLF and (b) detecting the presence of a EDLF; wherein the presence of said epitope is indicative of a carcinoma. The antibody-epitope complex may be detected by Western blot, radioimmunoassay, ELISA (enzyme linked immunosorbent assay), "sandwich" immunoassay, immunoprecipitation assay, precipitation reaction, gel diffusion precipitation reaction, immunodiffusion assay, agglutination assay, complement-fixation assay, immunohistochemical assay, fluorescent immunoassay, and protein A immunoassay. The sample may be sample is a tissue biopsy, lymph, urine, cerebrospinal fluid, amniotic fluid, inflammatory exudate, blood, or serum.

The anti-EDLF antibodies thereof may be used in diagnostic methods for detecting the presence or absence of EDLF, wherein the presence of the antigen is indicative of eclampsia, preeclampsia, and/or intraventricular hemorrhage. The diagnostic methods may be used with patients at risk of eclampsia, preeclampsia, and/or intraventricular hemorrhage or patients without symptoms.

The antibodies which selectively bind a EDLF may be recombinant. The fragments of antibodies which selectively bind a EDLF may be a Fab, Fab', F(ab')2, Fv, CDR, paratope, or portion of an antibody that is capable of binding the antigen. The antibodies which selectively bind a EDLF may be chimeric, humanized, anti-idiotypic, single-chain, bifunctional, or co-specific. The antibodies which selectively bind a EDLF may be or fragment is conjugated to a label, including but not limited to a chemiluminescent label, paramagnetic label (*e.g.,* aluminum, manganese, platinum, oxygen, lanthanum, lutetium, scandium, yttrium, or gallium), an MRI contrast agent, fluorescent label, bioluminescent label, or radioactive label.

Additionally, anti-EDLF antibodies thereof, may be attached to a solid support (*e.g.,* bead, test tube, sheet, culture dish, or test strip) such as an array.

The method may comprise imaging a EDLF by positron emission tomography (PET), CCD low-light monitoring system, x-ray, CT scanning, scintigraphy, photo acoustic imaging, single photon emission computed tomography (SPECT), magnetic resonance imaging (MRI), ultrasound, paramagnetic imaging, and endoscopic optical coherence tomography.

EDLF may be used as an eclampsia, preeclampsia, and/or intraventricular hemorrhage biomarker. Detection of the EDLFs in a biological sample, such as a subject's serum, may be performed by means of the anti-EDLF antibody or fragment thereof. For example, a biological sample (*e.g.,* serum or placenta sample) is obtained from a subject, then EDLF is measured (*e.g.,* by ELISA or PCR), and compared with corresponding samples from normal subjects. Measuring methods include any method of nucleic acid detection, for example in situ hybridization using antisense EDLF DNA or cRNA oligonucleotide probes, ultra-high throughput sequencing, nanostring technology, microarrays, rolling circle amplification, proximity-mediated ligation, PCR, qRT-PCR ChIP, ChIP-qPCR, or EDLF-binding antibodies. Comparatively high levels of EDLF indicate the presence and/or severity of eclampsia, preeclampsia, and/or intraventricular hemorrhage.

The anti-EDLF antibodies thereof, may be used in SQUID (Superconducting Quantum Interference Device) techniques for diagnostic methods. The SQUID technique comprises attaching nanoparticles of iron oxide to antibodies, which are then injected into the patient. See, *e.g.,* Hao, et al. (2010) Journal of Physics 43: 474004. In a SQUID method, the patient is then surrounded with sensitive magnetic coils in a superconducting quantum interference device (SQUID). A magnetic field is generated and all of the metal nanoparticles align in one direction. When the magnetic field is broken, the nanoparticles emit an electromagnetic signal as they relax back into their original state. By measuring the strength of the signal, one may tell how many metal particles, and therefore how much EDLF, may be present, and where in the patient the EDLF is located. See, *e.g.,* Shao, et al. (2010) Beilstein Journal of Nanotechnology 1: 142-154.

### Samples and Procurement of Samples

The samples used in the methods described herein may be taken from a subject (patient) include but are not limited to a blood, serum, plasma, placenta, or any combination thereof. Prior to be subjected to the diagnostic assay, the sample can optionally be diluted with a suitable diluent.

Numerous well known tissue or fluid collection methods can be utilized to collect the biological sample from the subject in order to determine the level of DNA, RNA and/or polypeptide of the marker of interest in the subject. Examples of tissue or fluid collection methods include, but are not limited to, fine needle biopsy, needle biopsy, core needle biopsy and surgical biopsy (*e.g.,* brain biopsy), and lavage. Regardless of the procedure employed, once a biopsy/sample is obtained the level of the marker may be determined and a diagnosis can thus be made.

### Detection of EDLF

The invention provides a method for detecting EDLF of this invention in a biological sample, comprising: contacting a biological sample with an antibody specifically recognizing a EDLF according to the present invention and detecting said interaction; wherein the presence of an interaction correlates with the presence of a EDLF in the biological sample.

EDLF described herein are non-limiting examples of markers for diagnosing a disease and/or an indicative condition. Each marker of the present invention may be used alone or in combination, for various uses, including but not limited to, prognosis, prediction, screening, early diagnosis, determination of progression, therapy selection and treatment monitoring of an eclampsia, preeclampsia, and/or intraventricular hemorrhage.

### ASSAYS

The EDLFs, antibodies and antigen-binding fragments that bind the EDLF, may be used in assays to qualitatively or quantitatively detect and analyze markers in a sample. For example, the EDLFs, antibodies and antigen-binding fragments that bind the EDLF, may be used in a nanowire biosenor assay to qualitatively or quantitatively detect and analyze markers in a sample. For example, the anti-EDLF antibody may be affixed to a nanowire which changes its conductivity when the anti-EDLF antibody binds an EDLF in biological sample. Further, an anti-digoxin antibody may be affixed to a nanowire which changes its conductivity when the anti-digoxin antibody binds digoxin in biological sample. The nanowire may be arranged in an array. The nanowire may be coupled to a chip. For example, a method for detecting digoxin may comprise obtaining a biological sample, optionally a blood sample, contacting the biological sample, optionally a blood sample, with a chip comprising a nanowire comprising an anti-digoxin antibody, and measuring the conductivity, wherein a change in conductivity is indicative of the presence of digoxin. The method may further comprise measuring the amount of digoxin present in the sample based on the change in conductivity.

For example, the EDLFs, antibodies and antigen-binding fragments that bind the EDLF, may be used in immunoassays to qualitatively or quantitatively detect and analyze markers in a sample. This method comprises providing an antibody specifically binds to a EDLF; contacting a sample with the antibody; and detecting the presence of a complex of the antibody bound to the marker in the sample.

An EDLF may be detected and/or quantified using any of a number of well recognized immunological binding assays. Useful assays include, for example, an enzyme immune assay (EIA) such as enzyme-linked immunosorbent assay (ELISA), a radioimmunoassay (RIA), a Western blot assay, or a slot blot assay. See, *e.g.,* U.S. Patent Nos. 4,366,241; 4,376,110; 4,517,288; and 4,837,168. Generally, a sample obtained from a subject can be contacted with the antibody specifically binds the EDLF.

Optionally, the antibody can be fixed to a solid support to facilitate washing and subsequent isolation of the complex, prior to contacting the antibody with a sample. Examples of solid supports include but are not limited to glass or plastic in the form of, *e.g.,* a microtiter plate, nanowire, a stick, a bead, or a microbead. Antibodies may be attached to a solid support.

After incubating the sample with antibodies, the mixture is washed and the antibody-marker complex formed may be detected. This can be accomplished by incubating the washed mixture with a detection reagent. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations. Usually the assays will be carried out at ambient temperature, although they can be conducted over a range of temperatures (*e.g.,* 10°C-40°C).

The immunoassay can be used to determine a test amount of a marker in a sample from a subject. First, a test amount of a marker in a sample may be detected using the immunoassay methods described above. If a marker is present in the sample, it will form an antibody-marker complex with an antibody specifically binds the marker under suitable incubation conditions described above. The amount of an antibody-marker complex can optionally be determined by comparing to a standard. As noted above, the test amount of marker need not be measured in absolute units, as long as the unit of measurement can be compared to a control amount and/or signal. Several immunoassays are known in the art and the EDLFs, and antibodies specific for said antigens described herein may be used in such immunoassays including but not limited to radioimmunoassay (RIA), enzyme linked immunosorbent assay (ELISA), magnetic immunoassay, immunoblot, Western blot, immunoprecipitation assays, immunohistochemical analysis, and fluorescence activated cell sorting (FACS). See Wild, (2008) [Ed.] The Immunoassay Handbook [3rd Ed.] Elsevier.

### RADIO-IMAGING METHODS

The EDLFs, antibodies and antigen-binding fragments that bind the EDLF, may be used in radio-imaging methods to diagnosis eclampsia, preeclampsia, and/or intraventricular hemorrhage. These methods include but are not limited to, positron emission tomography (PET) single photon emission computed tomography (SPECT). Both of these techniques are non-invasive, and can be used to detect and/or measure a wide variety of tissue events and/or functions, such as detecting eclampsia, preeclampsia, and/or intraventricular hemorrhage for example. SPECT may optionally be used with two labels simultaneously. See U.S. Patent No. 6,696,686.

All publications (*e.g.,* Non-Patent Literature), patents, patent application publications, and patent applications mentioned in this specification are indicative of the level of skill of those skilled in the art to which this invention pertains. All such publications (*e.g.,* Non-Patent Literature), patents, patent application publications, and patent applications are herein incorporated by reference to the same extent as if each individual publication, patent, patent application publication, or patent application was specifically and individually indicated to be incorporated by reference.

Although methods and materials similar or equivalent to those described herein may be used in the invention or testing of the present invention, suitable methods and materials are described herein. The materials, methods and examples are illustrative only, and are not intended to be limiting.

The invention now being generally described, it will be more readily understood by reference to the following examples, which are included merely for purposes of illustration of certain aspects and embodiments of the present invention, and are not intended to limit the invention.

### EXAMPLES

### EXAMPLE 1

### Placental homogenate preparation.

For these initial studies placentas were obtained from both normal and preeclamptic pregnancies immediately after delivery and a full thickness cut (∼2cm x 2cm x 2cm) was removed, snap frozen in liquid nitrogen and stored at -80°C until later processing and assay. Placental pieces were shaved into flakes using a surgical blade and tissue flakes were placed into a Sartorius Mikro Dismembrator stainless steel cylinder along with 15 stainless steel balls. The entire cylinder, including contents, was submerged in liquid nitrogen for 4-5 minutes. After the thorough freezing of the cylinder contents, the cylinder was placed in the Sartorius ball mill and shaken at 2000 rpm for 10 minutes. The process of submersion and shaking was repeated until the contents became a fine powder. The placental homogenate was transferred from the cylinder to a 50 mL conical tube and the volume was brought up to 5 mL by adding deionized H2O. To remove protein, two volumes of methanol (10 mL) were added gradually to the homogenate while the mixture was vortexed continuously for 5 minutes. The placental sample mixture was then centrifuged for 10 min at 4000 rpm to remove the precipitated proteins. The supernatant was transferred to a new conical tube and dried down overnight in vacuo to remove residual methanol. Then, the volume was brought back to the original volume of 5 mL using deionized H₂O. The placental homogenate was stored at -80°C for further processing and assay.

### Placental explant culture and conditioned medium collection

For these studies placentas from uncomplicated pregnancies were obtained immediately after delivery and 4-5 small tissue pieces (∼5mm x 5mm x 5mm) were cut off from the inner fetal side. The tissue pieces were dissected into tiny pieces with sterilized scissors. Any visible clots and blood vessels were removed with sterilized tweezers. The remaining villi were washed repeatedly with PBS to remove blood from the intervillous space. Villous tissue of ∼5 mg/well was then patted dry by sterilized paper tower and incubated in a 6-well cell culture plate with 5 mL of serum-free DMEM (Gibson, Grand Island, NY, USA) containing 100 U/mL penicillin, 100 µg/mL streptomycin and 0.25 µg/mL amphotericin B (Sigma, St. Louis, MO, USA) per well for 48 hours at 37°C in an incubator gassed with 95% air and 5% CO₂.

Ketoconazole (Sigma, St. Louis, MO, USA, 1 µM, 2 µM, 5 µM, 10 µM, 20 µM, 17α-hydroxyprogesterone (Sigma, St. Louis, MO, USA, 200 nM, 500 nM, 1 µM, 2 µM) or pregnenolone (Steraloids Inc., Newport, RI, USA, 2 µM), human tumor necrosis factor alpha (TNF-α, Sigma, St. Louis, MO, USA, 1 nM, 2 nM, 5 nM, 10 nM, 20 nM) or hydrogen peroxide (Fisher Scientific, Fair Lawn, NJ, USA, 1 nM, 5 nM, 10 nM, 20 nM) were added to the tissue culture individually at the beginning of incubation. Time course experiments (6 hours, 12 hours, 24 hours, 36 hours and 48 hours) were performed to study the effect of the potential substrate 17α-hydroxyprogesterone. For the low O₂ culture experiments, the culture plates were placed in a portable air chamber (Billups-Rothernberg, Del Mar, CA, USA) flushed daily with a gas containing 2% oxygen, 5% carbon dioxide, 93% nitrogen, (Airgas AcuGrav®, Salt Lake City, UT, USA) for up to 48 hours. The chamber was housed inside a regular incubator to maintain the 37°C. At the end of incubation, samples of the culture medium were collected in 15 mL conical tubes and any residual villi were removed by centrifuging at 4000 RPM for 2 min. The supernatant was stored at -80°C as conditioned medium until later processing and assay.

### Digoxin-immune antibody radioimmunoassay (RIA)

After the homogenization and tissue culture processes, homogenate and conditioned media samples were collected and assayed by radioimmunoassay. Digibind (GlaxoSmithKline, Research Triangle Park, NC, USA) was used as the primary antibody anti-EDLF/ouabain and a rabbit anti-sheep immunoglobulin (IgG) Fab fragment antibody (Jackson ImmunoResearch Laboratories, Inc., West Grove, PA, USA) was used as the secondary antibody. Digifab (Protherics/BTG, London, England) may also be used as a primary antibody in the assay and is expected to generate similar results due to the bioequivalence of Digifab and Digibind. Digibind and Digifab represent the Fab fragments of an anti-digoxin antisera produced in sheep and used therapeutically to counteract digoxin overdose in humans. Tritiated ouabain (30.0 Ci/mmol, Perkin Elmer, Boston, MA, USA) was used as a tracer. Cold ouabain solutions at graded concentrations were used as standards. A 100 µL aliquot of specimen or standard ouabain solution (50 nM, 0.1 µM, 0.2 µM, 0.5 µM, 1 µM, 3 µM), plus 50 µL of a 2.22 x 10-8 M tritiated ouabain solution, 300 µL of 1.8 µg/mL Digibind solution, 60 µL of 2.12 x 10-7 M 2° Ab solution and 10 µL of 10 mM pH 7.4 Tris buffer were combined and mixed well, then allowed to incubate at room temperature overnight to allow antigen-antibody binding. For the assay of conditioned media, because samples contained DMEM medium, 100 µL of DMEM were added to the reaction solutions for each standard and 100 µL of deionized H2O were added to reaction solutions for specimens to bring them to the same volume. EDLF in the several conditioned media/homogenate specimens or standard cold ouabain in the calibrating solutions competed with the labeled ouabain for Digibind and then the secondary (2°) antibody was added to bind to the primary (1°) antibody-antigen complex to decrease its solubility. After the overnight incubation, 563 µL of 16% polyethylene glycol (PEG)-6000 (Calbiochem, La Jolla, CA, USA) was added to each reaction solution to precipitate the antibody-antigen complex. After centrifugation at 13,200 rpm for 30 min, the supernatant was discarded and the pellet was resuspended in 500 µL of 0.05 M phosphate buffer (pH 7.0). Then 4 mL of EcoscintTM, a scintillation fluid (National Diagnostics, Atlanta, GA, USA), was added to the resuspended solution, and the mixture was measured by scintillation counter to determine EDLF concentration. All individual specimens were assayed in duplicate.

### Progesterone experiment

In order to insure that results of DigoxinAB RIA represented EDLF levels, we tested the effect of progesterone, the main placental sterol, on the assay. A volume of 20 µL progesterone solution at graded concentrations (final concentration 1.00 x 10⁻⁹ M, 1.00 x 10⁻⁸ M and 1.00 x 10⁻⁷ M), 100 µL of deionized H2O, 100 µL of conditioned medium sample, 50 µL of tritiated ouabain solution, 300 µL of Digibind solution, 60 µL of 2° Ab solution and 10 µL of 10 mM pH 7.4 Tris buffer were combined and mixed well. Reaction solutions containing 20 µL of deionized H₂O instead of progesterone solution were used as negative controls. Mixture solutions were incubated, precipitated and analyzed as described in DigoxinAB RIA procedure.

### Lipid hydroperoxide and TNFα determination assays

Conditioned media from placental explants cultured with hydrogen peroxide or under low O₂ conditions were collected and assayed by a Lipid Hydroperoxide (LPO) Assay Kit (Cayman Chemical, Ann Arbor, MI, USA) and a human TNF-alpha Quantikine ELISA Kit (R&D Systems, Minneapolis, MN, USA) following the manufacturers' methods to determine levels of lipid oxidation.

### Rubidium (Rb⁺) uptake graphite furnace atomic absorption spectrometry (GFAA)

This represents a functional bioassay of EDLF. Conditioned media containing EDLF released from cultured placental tissues were assayed by DigoxinAB RIA as described above to determine apparent EDLF concentration. Concomitantly, the same samples were assayed by cell Rb⁺ uptake using a graphite furnace atomic absorption instrument (GFAA, model 4100Z, Perkin Elmer, Waltham, MA, USA) that measures [Na⁺,K⁺]ATPase-dependent Rb⁺ transport into fresh human red blood cells (RBCs). Ouabain was used in this assay to achieve complete inhibition of the [Na⁺,K⁺]ATPase activity. Blood was collected from non-pregnant healthy volunteers into EDTA containing tubes, left to stand at room temperature for one hour and centrifuged at 4000 rpm at 4°C for 10 min to remove the plasma. The remaining RBCs were washed with two volumes (10 mL) of RbCl buffer (containing NaCl 135 mmol/L, RbCl 6.73 mmol/L, NaH2PO4 8.10 mmol/L, Na2HPO4 1.27 mmol/L and MgCl2 1.0 mmol/L, pH 7.45, omitting K+) three times vortexing for 5 min before recollecting the cells by centrifuging at 4000 rpm at 4°C for 10 min. After washing, a 10% hematocrit (Hct) RBC solution was prepared and 800 µL of this 10% Hct RBC solution, 100 µL of conditioned media sample or control (ouabain at 1.00 x 10-3 M final concentration) without and with 100 µL of Digibind solution (1.00 x 10-6 M final concentration) were mixed in an Eppendorf tube and rocked in an incubator at 37°C at 220 rpm for 45 min to allow Rb⁺ ion uptake into the cells. After incubation, each bioassay solution was centrifuged at 4000 rpm for 10 min to isolate the RBCs from the RbCl buffer. The RBCs were then washed by adding 1 ml of ice cold washing buffer (containing choline chloride 149 mmol/L, MgCl2 1.0 mmol/L, MOPS 5.88 mmol/L, Tris 2.12 mmol/L, pH 7.40) and centrifuged again to remove residual extracellular Rb⁺. This washing process was repeated 3 times. Cells were lysed by addition of deionized H2O, centrifuged to remove cell ghosts (4000 rpm) and stored at -80°C overnight. Rb⁺ uptake into the cells was then measured by standard operation of a GFAA instrument to determine the ability of EDLF to inhibit [Na⁺,K⁺]ATPase-mediated ion transport. Before the GFAA step, 10 µL of the isolated cytosol from the RBCs were diluted to 500 µL with deionized H2O from which the autosampler of the GFAA injected 10 µL for Rb⁺ ion quantitation. All samples were assayed in triplicate.

### Statistical Analyses

Results are reported as the mean + 1 SEM. Comparisons of the EDLF measured by DigoxinAB RIA with EDLF measured by the GFAA Rb⁺ ion uptake assay were carried out by means of Pearson's Product Moment Correlation analysis. Effects of time or concentration on EDLF release from placental tissue were analyzed by ANOVA with post hoc Dunnett's pair-wise comparisons. Comparisons involving two groups were carried out by two tailed Student's t-test. A p-value of <0.05 was considered significant.

### RESULTS

A SP inhibitor or EDLF exists in placental homogenates, with higher amounts observed in placenta from women having PE. The placenta produces an EDLF. It was determined whether placental explants were capable of releasing EDLF and second any such material was tested if it could be recognized both by its ability to inhibit the SP and to interact with a digoxin antibody Fab fragment (DigoxinAB) to complex EDLF. To accomplish this, the first undertaking was to develop an immunoassay using the digoxin antibody Fab fragment, Digibind, that has been shown to bind EDLF.

### EDLF specific immunoassay employing Digibind

A clinical trial, termed the DEEP Trial found that preeclamptic women who had increased serum EDLF levels experienced clinical benefit with Digibind compared with placebo treatment. Graves, et al. (2007) Frontiers in Bioscience 12: 2438-2446. Moreover, recent research also points to EDLFs present in both serum and placenta from women with PE being bound and inactivated by Digibind. Menezes, et al. (2003) Amer J Hypertens 16: 1062-1065. Digibind may recognize and bind the active EDLF found in PE, therefore a radioimmunoassay (RIA) using Digibind as the primary antibody may be used to detect PE. This assay may serve as a probe to identify women having observable serum EDLF and which consequently should respond favorably to DigoxinAB (anti-digoxin antibody) treatment, *i.e.* a theranostic test to predict which women will benefit from treatment with DigoxinAB. Using tritiated ouabain as tracer, a standard curve was developed using graded concentrations of non-radiolabeled ouabain as standards.

Using the above DigoxinAB RIA, EDLF has been measured in the serum of pregnant women with PE. Some women had substantially higher concentrations of EDLF than others. This assay was also successfully applied to the measurement of EDLFs in placental homogenates.

Using this novel DigoxinAB RIA, 11 conditioned media specimens assayed by both DigoxinAB RIA and by SP inhibition of red cell Rb uptake demonstrated that there was a significant correlation between these two assays (Figure 1, R=0.69, p=0.019).

Data indicated that protein-depleted placental homogenates from women with PE appeared to have higher EDLF levels than levels found in PE sera, sometimes much higher. This observation suggested that placenta might be a source of EDLF. As was found with PE serum, some pregnant women had substantially higher placental concentrations of EDLF than others. Moreover, comparison of EDLF concentrations in placental homogenates from women with PE with those from women with uncomplicated pregnancies showed that PE placentas had increased tissue EDLF levels and this difference between normal and PE homogenates was still significant even with sequential dilutions of the specimens (Figure 2, neat serum, PE 32.88 ± 14.63 vs CTL 7.44 ± 1.15 x 10⁻⁸ M ouabain equivalents, p=0.0002; 1:2 dilution, PE 20.76 ± 11.29 vs CTL 6.28 ± 0.85 x 10⁻⁸ M ouabain equivalents, p=0.002; 1:3 dilution, PE 16.96 ± 9.12 vs CTL 5.04 ± 0.50 x 10⁻⁸ M ouabain equivalents, p=0.002; 1:4 dilution, PE 15.61 ± 11.47 vs CTL 4.17 ±0.54 x 10⁻⁸ M ouabain controls, p=0.02). This study and the previous comparing the two assays provide evidence that placenta was a source of EDLF and that the DigoxinAB RIA employing Digibind as the antibody appropriately measured EDLF in placenta and had adequate sensitivity.

Finally, the EDLF secreted into the culture media of freshly explanted normal human placenta could also be assessed. EDLF concentrations in the media from normal placental culture were in the range of those found in PE sera.

### EDLF synthesis and synthetic pathway in placenta

In addition to identifying placenta as a source of EDLF in PE, it was also of interest to provide greater understanding of its tissue production, including an indication of what pathways may be involved in its production. EDLF is a sterol and consequently its synthetic pathway may share steps with the steroid synthetic pathway. There are known substrates of these pathways and also some agents that can block some of the enzymes involved.

To determine if EDLFs required steps in the steroid synthesis pathway, experiments using ketoconazole, an agent that blocks steroid synthesis were conducted. Aguananne, et al. (2011) Am J Perinatol 28: 509-514; Graves, et al. (1993) J Cardivasc Pharmacol 22(S2): S54-57. When this drug was applied to explanted placental tissue, it caused a marked reduction in EDLF production and release into the culture media in a dose-dependent manner. In the fetal tissues of normal human placenta there was a significant decrease in EDLF production in response to ketoconazole (Figure 3A, with graded increasing concentrations of ketoconazole respectively, (1.0 µM) 7.99 ± 13.21, (2.0 µM) 5.58 ± 8.91, (5.0 µM) 1.60 ± 1.62, (10.0 µM) 1.47 ± 1.64, (20.0 µM) 1.25 ± 1.43 vs control levels 17.78 ± 33.21 x 10⁻⁸ M RIA ouabain equivalents; ANOVA of the fractional change, p<0.001), whereas the maternal tissues showed substantially less production under these conditions and little change or if anything a small increase in EDLF production with 48 hours of ketoconazole treatment compared with untreated tissue (Figures 3B, with graded increasing concentrations of ketoconazole respectively, (1.0 µM) 1.90 ± 2.05, (2.0 µM) 1.79 ± 1.96, (5.0 µM) 1.83 ± 2.03, (10.0 µM) 1.73 ± 1.82, (20.0 µM) 2.46 ± 2.60 vs control 1.23 ± 1.41 x 10⁻⁸ M RIA ouabain equivalents; ANOVA of the fractional change, p=0.51). Basal fetal EDLF levels were higher than basal maternal levels (p=0.03). These results confirm that ketoconazole has a pronounced inhibitory effect on EDLF production and release.

In an effort to assess whether EDLF production involves enzymes used in steroid synthesis, the inventors tested actors which may serve as precursors or intermediates of steroid synthesis and hence may increase EDLF synthesis and release. Two possible steroid precursors were tested and found to identify steps common to both synthetic pathways. Graded concentrations of 17α-hydroxyprogesterone (17P) were applied to placental tissue culture. This particular steroid was chosen because placenta is reported to lack a 17-hydroylase enzyme. Figure 4 shows that 48 hours of 17P treatment resulted in elevated levels of EDLF released into the culture media in a dose-dependent manner with increasing concentrations of 17P respectively, (0.20 µM) 3.60 ± 1.06, (0.50 µM) 3.76 ± 0.84, (1.0 µM) 4.40 ±1.12, (2.0 µM) 4.91 ± 1.52 vs control 2.28 ± 0.41 x 10⁻⁸ M ouabain equivalents; ANOVA, p=0.003, with concentrations 1.0 and 2.0 µM being significantly greater than control, Dunnett's test). In order to further characterize its role in EDLF synthesis, time-dependent experiments were also carried out using an optimal concentration (2.0 µM) of the 17P. Control specimens in the absence of 17P showed that EDLF in the medium increased with longer incubation time (Figure 5A, n=5, 6 hours 8.76 ± 2.56, 12 hours 23.70 ± 6.92, 24 hours 39.32 ±14.76; 36 hours 67.59 ± 26.89, 48 hours 81.38 ± 18.77 x 10⁻⁸ M ouabain equivalents, ANOVA, p<0.001). Moreover, addition of 17P to the culture media enhanced the accumulation of EDLF in culture in a time dependent manner with significantly more EDLF produced at each time point compared with the control (Figure 5B, n=5, 6 hours 12.60 ± 3.81, 12 hours 28.30 ± 6.30, 24 hours 55.39 ± 33.62, 36 hours 95.00 ± 40.44, 48 hours 132.43 ± 74.37 x 10⁻⁸ M ouabain equivalents, p<0.001 ANOVA for trend and p=0.03 for comparison of the effect of the AUC of 17P vs the AUC with no 17P). These results establish that 17P regulates placental EDLF synthesis.

Experiments with pregnenolone, were conducted an early precursor of many steroids. These experiments showed a marked reduction in placental EDLF production after 6 hours of exposure to 2 µM pregnenolone. EDLF concentrations in the conditioned media were: n=5, 6 hours 325.6 + 61.0 x 10⁻⁸ M; 12 hours 136.1 + 27.7 x 10⁻⁸ M; 24 hours 148.4 + 15.1 x 10⁻⁸ M; 36 hours 113.6 + 14.4 x 10⁻⁸ M; 48 hours 48.5 ± 7.4 x 10⁻⁸ M; p<0.0001 ANOVA, all other values were significantly reduced compared with the 6 hour value, p<0.05, Dunnett's test. See Figure 6. For this particular agent earlier time points were also assessed. Conditioned media was collected prior to pregnenolone treatment and then after 3 hour and 6 hour exposure to 2 µM pregnenolone. The abundance in the conditioned media didn't change appreciably over these three time points although the results were variable. Overall there was a slight but insignificant decline in EDLF (n=5): Pre-pregnenolone 81.3 + 13.7 x 10⁻⁸ M, 3 hours 77.4 ± 25.0 x 10⁻⁸ M, 6 hours 76.8 + 35.0 x 10⁻⁸ M ouabain equivalents, p>0.05.

### Regulation of EDLF release from placenta

PE is attended by many abnormalities. Among those considered mechanistically important are placental hypoxia, increased production of reactive oxygen species, and greater local and circulating levels of pro-inflammatory cytokines.

Because previous studies have suggested that hypoxia contributes to the development of PE, placental tissues were cultured under low O₂ conditions in comparison to tissues cultured under 21% O₂, then measured EDLF levels in the media and compared treatment to control. Figures 7A and 7B show that hypoxia minimally stimulated EDLF production and release into the culture media after both 24 hours and 48 hours incubation (24 hr: 2% O₂ 7.00 ± 1.62 vs 21% O₂ 5.94 ± 1.09 x10⁻⁸ M ouabain equivalents, p=0.028 Wilcoxon test; 48 hr: 2% O₂ 3.45 ± 0.66 vs 21% O₂ 2.92 ± 0.59 x 10⁻⁸ M ouabain equivalents, p=0.028, Wilcoxon). The changes were statistically significant or at the statistical p-value cut off.

Because oxidative stress has been shown to be increased in PE, thus hydrogen peroxide, a reactive oxygen species, was used to treat placental tissue in culture and assayed the media to reveal effects on EDLF release to the media. By analyzing specimens from six individual placentas treated with graded concentrations of H₂O₂, we found that 5 nM of H₂O₂ was the amount to induce maximal EDLF production and release. This concentration resulted in a near doubling of the quantity of released EDLF compared with the conditioned media from untreated placenta (Figure 8, 4.87 ± 1.57 vs 2.82 ± 0.60 x 10⁻⁸ M ouabain equivalents, p=0.009). In the media of cultured placenta treated with higher concentrations (10 nM, 20 nM) of H₂O₂, EDLF levels were equal or slightly lower than those treated with 5 nM H₂O₂. These observations establish that the effect of H₂O₂ on EDLF production plateaus with higher concentrations having no further or even a deleterious effect perhaps due to tissue damage or even damage of EDLF.

Experiments using TNFα were conducted to test its effect on placental EDLF production. Assay data showed that after 48 hours of TNFα treatment, levels of EDLF released into the culture media had significantly increased and this effect was dependent on TNFα concentration (Figure 9): n=6, (1.0 nM) 2.41 ± 0.80, (2.0 nM) 2.75 ± 0.98, (5.0 nM) 2.76 ±0.80, (10.0 nM) 3.51 ± 0.69, (20.0 nM) 4.47 ± 0.99 vs no TNFα control 1.96 ± 0.64 x 10⁻⁸ M ouabain equivalents; p<0.001, with the two higher concentrations being significantly higher than control, p<0.05, Dunnett's test). These results establish that TNFα causes increased EDLF release in addition to TNFα mediating several other changes.

### Effects of hypoxia and oxidative stress on lipid perioxidation and TNFα release from human placenta.

As discussed herein, low O₂ tension potentially, and certainly low concentrations of H₂O₂, induced elevated EDLF levels. In order to further assess the role of these factors on placental cell membrane modification in PE, a lipid hydroperoxide immunoassay was used to quantify lipid peroxidation in placenta culture media treated with low O₂ or H₂O₂. Figure 10 summarizes the effects of low oxygen tension versus normal oxygen levels on lipid peroxides (n=6, normal O₂ 5.85 ± 3.11 vs low O₂ 10.30 ± 5.72 µM; p=0.01). Figure 11 shows that there are higher levels of lipid peroxides produced when placenta is exposed to graded concentrations of H₂O₂ (Figure 11, n=5, (1.0 nM) 6.62 ± 3.31, (5.0 nM) 9.17 ± 3.18, (10.0 nM) 11.43 ± 3.67, (20.0 nM) 13.13 ± 5.04 vs control 5.05 ± 2.69 µM lipid peroxides respectively; ANOVA, p=0.017, with the highest two concentrations being significantly different than control, p<0.05, Dunnett's test). However, levels of lipid peroxidation for 2% O₂ and 5 nM H₂O₂ were very comparable, the levels being only slightly higher for low O₂.

### The interaction of hypoxia and oxidative stress on placental TNFα production.

A TNFα immunoassay was used to measure TNFα concentrations in culture media of human placenta in response to either 48 hours low O₂ (2%) or 48 hours of 5 nM H₂O₂. Figure 12 shows that 48 hours low O₂ treatment induced more TNFα release than media incubated under normal 21% oxygen conditions (n=6, 126.80 ± 249.61 vs 19.01 ± 10.16 pg/mL; p=0.03, Wilcoxon test) whereas, as shown in Figure 13, there was not a significant increase of TNFα release with adding increasing graded concentrations of H₂O₂: n=5, (1.0 nM) 18.61 ± 13.60, (5.0 nM) 14.95 ± 6.39, (10.0 nM) 15.83 ± 6.63, (20.0 nM) 13.50 ± 3.43 vs control 15.13 ± 3.98 pg/mL; p=0.90).

### DISCUSSION

EDLFs are potential hypertensinogenic factors in essential and experimental hypertension, and have also been found to be increased in the setting of PE. EDLF increase peripheral vascular resistance while potentially maintaining cardiac output. The placental source of EDLF explains the rapid disappearance of EDLFs from the maternal circulation after delivery and a rapid resolution of maternal hypertension post partum. The data presented herein demonstrate that human placenta is not only an enriched source of EDLF but that it synthesizes and releases EDLF into conditioned media. The presence of these secreted EDLFs has been demonstrated by both an antibody assay and by a functional assay measuring sodium pump inhibition. The DigoxinAB RIA is a useful assay for determining whether a woman experiencing PE may be effectively treated with Digibind or Digifab.

Studies applying both DigoxinAB RIA and GFAA established that ketoconazole is an inhibitor of EDLF production in placenta whereas 17α-hydroxyprogesterone directly or indirectly regulates EDLF synthesis. These effects were dose-dependent.

The experiments with pregnenolone also establish that the steroid synthetic pathway is involved in placental the production of EDLF. Application of pregnenolone to explanted placental tissues reduces EDLF production. Thus, EDLFs are synthesized and secreted by human placenta.

Both 24 hours and 48 hours hypoxia (2% O₂) treatment appeared to induce modest increases in EDLF production and release from placenta in tissue culture.

Clinical evidence shows that markers of oxidative stress in placenta are elevated in PE. The effect of H₂O₂ on EDLF production was tested and found that a low concentration of H₂O₂ could stimulate EDLF production and release from healthy human placental tissue placed in culture. The changes in response to H₂O₂ were much more pronounced than those observed with low O₂. The ROS findings are consistent with the hypoxia findings discussed above and further demonstrate that these abnormalities associated with PE can regulate EDLF synthesis and release through one or more of the complex pathways they initiate.

Endothelial dysfunction is a central pathophysiologic feature of PE. Altered endothelial function involves, among other things, an exaggerated inflammatory response in PE, including increased circulating cytokine levels of TNFα. TNFα stimulates human placental EDLF production and release in a dose dependent manner. Therefore, reduction of TNFα (*e.g.,* via anti-TNFα, anti-TNFa receptor or other drugs or biologics that inhibit production of or reduce efficacy of TNFα) will reduce placental production of EDLF in the setting of PE and thereby reduce symptoms of PE caused directly or indirectly by EDLF. Accordingly, anti-EDLF antibody may be used in immunoassays to detect the presence of EDLF and quantify the levels of EDLF. Further, elevated levels of EDLF are indicative of eclampsia and preeclampsia as well as responsiveness to anti-digoxin antibody or antibody fragment thereof therapy.

### EXAMPLE 2

### Theranostic Assay Summary

Identification of pregnant women whose pregnancies are complicated by preeclampsia (PE) and who have elevated levels of these EDLFs allows for the appropriate use of an antibody Fab fragment therapy which binds these same factors and reduces their effects.

### Background

Substantial research over several decades has provided a vast amount of research on endogenous inhibitors of the sodium pump, sometimes called endogenous digitalis-like factors (EDLFs). Goto, et al. Pharmacol Rev 1992;44:377-99; Haddy FJ, Buckalew VM Jr. Endogenous digitalis-like factors in hypertension. In Brenner BM and Laragh JH (eds) Hypertension: Pathophysiology, Diagnosis, and Management. Raven Press, New York, 1995; pp 1055-106; Graves SW, Williams GH. Ann Rev Med 1987;38:433-444; Graves SW. Curr Opinion Nephrol Hypertens 1994;3:107-111. Increased levels of these factors have been implicated in many hypertensive disorders. The reasons for these EDLFs being increased is unclear but there is overwhelming data to support them being increased in serum in human essential hypertension, many secondary forms of human hypertension, in many experimental animal models of hypertension and in preeclampsia, a hypertensive disorder of pregnancy. Graves & Williams J Clin Endocrinol Metab 1984; 59:1070-4; Graves Hypertension 1987; 10(S Pt 2):I-84-6; Graves Frontiers in Bioscience 2007;12:2438-2446.Evidence supports these factors being structurally like digoxin and other cardioactive sterols and demonstrating similar activities.

These factors can cause hypertension experimentally. Inhibition of the sodium pump (SP) in the vasculature leads to increased vasoconstriction and resultant blood pressures. These effects can be prevented or reversed by some antibodies directed at digoxin, including two anti-digoxin antibody Fabs used to treat digoxin toxicity. Smith, et al. N Engl J Med 1982;307:1357-62; Krep, et al. Am J Hypertens 1995; 9:39-46.

### DEEP Study

The DEEP trial was a multicentered, double blinded registered clinical trial of the digoxin immune Fab (DIF) Digibind. Its rationale was that women with preeclampsia have higher levels of EDLF which mediates features of PE. Graves SW. The sodium pump in hypertension. Curr Opinion Nephrol Hypertens 1994;3:107-111; Graves SW, Williams GH. An endogenous ouabain-like factor associated with hypertensive pregnancies. J Clin Endocrinol Metab 1984; 59:1070-4; Graves SW. The possible role of digitalis-like factors in pregnancy-induced hypertension. Hypertension 1987; 10(S Pt 2):I-84-6.

Digoxin immune Fab (DIF) treatment can bind the EDLFs, blocking their actions and thereby reverse features of PE. Goodlin RC: Antidigoxin antibodies in eclampsia. N Eng J Med 1988;318:518-519; Adair, et al. Am J Hypertens 1997;10:11A ; Adair, et al. Am J Nephrol 1996;16:529-531. Specifically, preeclamptic recipients of digoxin immune Fab (DIF) showed significantly better renal function that preeclamptics receiving placebo. Moreover, digoxin immune Fab (DIF) treatment, as predicted, lowered circulating EDLF levels.

In the DEEP study these severe preeclamptic women demonstrated markedly higher levels of SP inhibition (the measure of EDLFs) compared with pregnant women having uncomplicated pregnancies. However, it was also observed that not all PE women had appreciable levels of EDLF present in their circulation. About 20% of the DEEP subjects had negligible levels of EDLF. In these women digoxin immune Fab (DIF) treatment would be anticipated to have no effect.

Those women who were EDLF negative and received digoxin immune Fab (DIF) did not display changes in maternal or fetal parameters. When EDLF positive women received digoxin immune Fab (DIF), they continued to show reductions in circulating EDLF and improved renal function, but with even more pronounced differences and even greater statistical significance despite fewer numbers. Additionally, many additional parameters showed improved differences and better statistical differences. For example, marked and statistically significant reductions in the rate of fetal intra-ventricular hemorrhage and maternal pulmonary edema in the EDLF-positive, digoxin immune Fab (DIF)-treated PE women were found. Digoxin immune Fab (DIF) treatment delayed delivery 24 hours and there was less antihypertensive use in these same women, although these differences were not statistically different.

EDLF positive PE women would likely show benefit with digoxin immune Fab (DIF) treatment, whereas EDLF negative PE women could be spared unnecessary and expensive treatment. This classification could be accomplished by an antibody based blood assay that measures EDLF positivity. The development of such a 'theranostic' assay would allow for the rationale application of digoxin immune Fab (DIF) treatment to only those individual PE women who would benefit from it.

### Development of a Theranostic

The inventors developed a radioimmunoassay (RIA) measuring EDLF that could be applied to serum from pregnant women. This has been accomplished using Digibind, this same digoxin antibody Fab used as a therapeutic, as the probe. Using tritiated ouabain as tracer, a standard curve was developed using graded concentrations of nonradiolabeled ouabain. A typical standard curve for this competitive binding assay is shown in Figure 14. Using this assay it is possible to measure EDLF in serum from PE women. Also, there is a correlation between the bioassay, measuring inhibition of sodium pump mediated Rb⁺ uptake and the RIA employing Digibind as the antibody.

A survey of monoclonal antibodies (Mab) raised against digoxin as the immunogen to find those that bind EDLFs-three Mab demonstrate a useable interaction with the EDLF and may be used in a theranostic assay. A sample standard curve is provided in Figure 15.

### EXAMPLE 3

Endogenous digitalis-like factors (EDLF) are elevated in women with preeclampsia, and the use of an anti-digoxin antibody Fab (DIF) in preeclamptic women remote from term reduces maternal blood pressure and preserves renal function. The objective here was to determine whether digoxin immune Fab (DIF) treatment in women with severe preeclampsia in association with positive EDLF in maternal serum improves maternal-perinatal outcomes.

### Study Design

This is a planned secondary analysis from a randomized, placebo-controlled, double blind study of digoxin immune Fab (DIF) in women with severe preeclampsia with positive EDLF status managed expectantly between 23 5/7 and 34 weeks gestation (19 women received placebo and 17 digoxin immune Fab (DIF)). Primary outcome variables were change in renal function (creatinine clearance, CrCl) and use of antihypertensives. Secondary outcomes were maternal and perinatal outcomes.

### Results

Women with positive EDLF who received digoxin immune Fab (DIF) had an attenuated decline in CrCl from baseline compared to placebo (-4.5±12.9 vs -53.2±12.6 mL/min, p=0.005). They also had a trend towards lower use of antihypertensives (41% vs 63%, p=0.12). Additionally, digoxin immune Fab (DIF) treated women had a lower rate of pulmonary edema (1/17 vs 6/19, p=0.035), lower rates of all neonatal intraventricular hemorrhage (IVH, digoxin immune Fab (DIF): 0/17 vs placebo: 5/19, p=0.015), and IVH in infants with birth weight <1250 g (digoxin immune Fab (DIF): 0/14 vs placebo: 5/11, p=0.012).

### Conclusion

In women with severe preeclampsia remote from term who are EDLF positive, the use of digoxin immune Fab (DIF) is associated with improved maternal and neonatal outcome. These findings suggest the need for a large multicenter trial evaluating the benefits of digoxin immune Fab (DIF) in management of women with severe preeclampsia and positive EDLF status remote from term.

The reported incidence of preeclampsia ranges from 3-5 % of all gestations. This incidence is expected to increase because of the rising prevalence of several risk factors for preeclampsia (PE) such as maternal obesity, gestational diabetes mellitus, chronic hypertension, advanced maternal age at time of pregnancy, and multi-fetal gestation. Barton & Sibai Obstet Gynecol 2008;112:359-372.

Pregnancies complicated by severe PE at < 34 weeks gestation are associated with high rates of maternal and perinatal complications, and the rates of these complications are dependent on gestational age at time of onset as well as on the type of management used (immediate delivery versus expectant management). Sibai & Barton Am J Obstet Gynecol 2007;196:514.e1-514.e9. Management of women with severe PE at < 34 weeks is aimed at keeping the mother and fetus safe, and delivery of a newborn that will survive and not require prolonged or intensive neonatal care. Recent studies suggested that expectant management is possible in a select group of women with severe PE between 24 and 34 weeks gestation, and that such management improves neonatal outcome, but is also associated with increased rates of maternal complications such as HELLP syndrome, pulmonary edema, deterioration in renal function, and eclampsia. Sibai & Barton Am J Obstet Gynecol 2007;196:514.e1-514.e9.

Endogenous digitalis-like factors (EDLF) represent a family of circulating inhibitors of the sodium pump (SP). Such SP inhibition causes direct vasoconstriction, and has been linked to an increased blood pressure in essential and experimental hypertension. Krep et al. Am J Hypertens 1995; 9:39-46; Soszynski et al. Am J Hypertens 1997;10:1342-48; Krep et al. Am J Hypertens 1995;8:921-7; Glatter et al. Am J Hypertens 1994;7:1016-25. EDLF is also elevated in the circulation of women with PE. Graves & Williams J Clin Endocrinol Metab 1984;59:1070-4; Gusdon et al. Am J Obstet Gynecol 984;15:83-85; Seely et al. J Clin Endocrinol Metab 1992; 74:150-6; Lopatin et al. J Hypertens 1999;17:1179-1187. Hopoate-Sitake et al. Reproductive Sci 2011;18:190-199. The addition of a specific commercially-available anti-digoxin antibody Fab *in vitro* reduced the inhibitory effects of EDLF on the SP. Krep et al. Am J Hypertens 1995; 9:39-46.

This same Fab has also been shown to have an antihypertensive effect in animal models of hypertension thought to be mediated by EDLF. Krep et al. Am J Hypertens 1995;8:921-7. Previously, administration of digoxin immune Fab (DIF) to women with PE reduced maternal blood pressure and preserved or improved renal function. Goodlin RC. N Engl J Med 1988; 318:518-9; Adair, et al. Am J Nephrol 1996;6(6):529-31; Adair, et al. J Perinatolog 2009;29:284-289. Indeed, these data led to a randomized, double-blind, placebo controlled trial (DEEP Trial) of digoxin immune Fab (DIF) in severe preeclamptic women. Adair, et al. Amer J Perinatol 2010;27:655-662 This study demonstrated a benefit from digoxin immune Fab (DIF) treatment on renal function in all women enrolled in the trial. The trial, however, found no benefit regarding pregnancy prolongation or improved maternal outcome. The primary analysis of the DEEP trial did not take into account the EDLF status of the women. It is possible, even likely, that digoxin immune Fab (DIF) is only efficacious in women who are positive for EDLF.

When circulating EDLF was subsequently measured at baseline in the 51 subjects enrolled in the DEEP trial, about 20% had no detectable circulating EDLF. Given the proposed mechanism of action (*i.e.* binding and inactivating EDLF) digoxin immune Fab (DIF) administration should have no effect in those women who did not have circulating EDLF, and their inclusion in the intent to treat analyses may have diminished the apparent effect of digoxin immune Fab (DIF) on preeclamptic women with measurable EDLF.

A planned secondary analysis from the DEEP trial in which digoxin immune Fab (DIF) effect was evaluated in women who were EDLF positive. The objective of this study was to determine whether digoxin immune Fab (DIF) treatment in women with severe PE in association with positive EDLF activity in maternal serum improves maternal and perinatal outcomes, and whether the effect is dependent on circulating EDLF levels at time of enrollment.

### MATERIALS AND METHODS

### Original Study Design and Sodium Pump Inhibition Assay of EDLF

A detailed description of the DEEP study has been previously published. Adair CD, Buckalew VM, Graves SW, Lam GK, Johnson DD, Saade G, Lewis DF, Robinson C, Danoff TM, Chauhan N, Hopoate-Sitake M, on behalf of the DEEP Study Group. Digoxin Immune Fab Treatment for Severe Preeclampsia. Amer J Perinatol 2010;27:655-662.Briefly, all participants were pregnant women who fulfilled the American College of Obstetricians and Gynecologists criteria for severe PE. Adair, et al. Amer J Perinatol 2010;27:655-662 IRB approval had been obtained at each study site and all subjects provided informed, signed consent prior to participation. Other eligibility criteria included a pregnancy between 23 weeks, 5 days and 34 weeks gestation and expected delivery of the fetus within 72 hours as judged by the primary physician. Adair, et al. Amer J Perinatol 2010;27:655-662 The intent of the original study was to test the efficacy of digoxin immune Fab (DIF), (Digibind, GlaxoSmithKline, Research Triangle Park, NC) on two primary endpoints: change in creatinine clearance as a measure of renal function and anti-hypertensive use as a measure of improvement or deterioration of hypertension. Women were randomized to digoxin immune Fab (DIF) or placebo which was given intravenously every 6 hours for up to eight total doses. Digoxin immune Fab (DIF) was administered to 24 of the 51 women included in the study. Patients, physicians and laboratory personnel were blinded to treatment arm and all clinical parameters including EDLF status were compiled prior to unblinding of the study. Treatment of PE in study patients, including use of antihypertensive drugs and timing of delivery was determined by clinical condition as judged by the primary physician.

As part of the original study, EDLF in plasma was measured by the plasma's ability to inhibit the SP of red blood cells obtained freshly from normal, non-pregnant volunteers. This assay, which measures SP-mediated uptake of Rb⁺ ion from an artificial medium into red cells in the presence and absence of inhibitor, has been described previously and validated in other studies. Zhen, et al. J Nutritional Biochem 2005;16:291-296.

If there is EDLF present, then less Rb⁺ is taken up into the cytosol of the RBCs. EDLF activity was determined in triplicate at baseline (prior to drug or placebo), and at 12, 24, and 48 hours (t = 0, 12, 24, 48 hr). Results of the Rb⁺ uptake assay at baseline were used to classify patients as being EDLF negative or EDLF positive. Among the 51 women enrolled in the original trial, samples for EDLF were available and evaluated in 46 subjects, 10 (22%) were negative and 36 (78%) positive. This secondary analysis focuses on the 36 women who were EDLF positive among whom 19 received placebo and 17 received digoxin immune Fab (DIF).

The primary outcome variables were the same as in the primary analysis, *i.e*. change in renal function (CrCl) and use of antihypertensives. Use of anti-hypertensive medications was defined as 1) first use of anti-hypertensive medication during the treatment phase or 2) an increase in anti-hypertensive medications during the treatment phase in subjects already on anti-hypertensive medications at the time of entry into the study or 3) delivery necessitated by persistent severe hypertension.

Secondary outcomes were clinical and laboratory markers of maternal (*e.g.* pulmonary edema, HELLP syndrome, blurred vision), fetal (*e.g.* persistent non-reassuring fetal status, fetal heart tracing abnormalities (including tachycardia, bradycardia, a decrease in beat-to-beat variability, or an abnormal pattern such as variable decelerations or late decelerations), and neonatal complications (*e.g.*, neonatal birth weights, respiratory distress syndrome and intraventricular hemorrhage (IVH)).

### Statistical Analysis

The analysis was performed by a contract research organization (Covance Inc) in a blinded fashion.

Continuous data were analyzed by ANCOVA with screening value, treatment group, gestational age and study site in the model or by logistic regression analysis with treatment, gestational age and center in the model. Categorical data were analyzed by Barnard Exact Test. Mehta, et al. Amer Statistician 1993;47:91-98. Chan Statistics Med. 1998;17:1403-1413. A p-value <0.05 was considered statistically significant. Change in CrCl was calculated as the difference in mL/min of treatment time minus the screening value.

### RESULTS

Table 1 compares the demographic characteristics at enrollment between the two treatment groups in the EDLF positive PE women. There were no significant differences between the women who received digoxin immune Fab (DIF) or placebo in any of the variables analyzed.

### Effect of digoxin immune Fab (DIF) on Circulating EDLF activity.

Digoxin immune Fab (DIF) treatment of EDLF positive women, when compared with the initial DEEP analysis, which included both EDLF positive and negative subjects, demonstrated larger and more statistically significant reductions in circulating EDLF levels as compared to pretreatment EDLF levels (Fig. 1). Adair, et al. Amer J Perinatol 2010;27:655-662. In the all subjects analysis, *i.e*. inclusion of both EDLF positive and negative subjects, only the 12 to 24 hours difference was significant (+11.0% SP activity recovery, p=0.03).

### Effect of digoxin immune Fab (DIF) on Primary Outcome Measures.

Figure 2A compares the effects of digoxin immune Fab (DIF) treatment to placebo on change in CrCl. EDLF positive, severe PE women receiving digoxin immune Fab (DIF) had a significantly smaller drop in CrCL from baseline as compared to placebo. In addition, renal function deterioration in the control group was positively related to the EDLF level (women with >30% SP inhibition p=0.032, Figure 2B). Compared to the all subjects analysis, the difference between treatment and placebo for use of antihypertensives was greater, although the differences did not reach statistical significance (EDLF positive: 41% digoxin immune Fab (DIF) vs 63% placebo, p=0.12; All subjects: 46% digoxin immune Fab (DIF) vs 52% placebo, p=0.4).

### Effect of digoxin immune Fab (DIF) on Secondary Outcomes

Table 2 compares latency period from study entry, gestational age at delivery, birth weight, changes in fetal heart rate (FHR) tracing, rate of non-reassuring FHR testing, neonatal respiratory distress syndrome, neonatal IVH and death between the two study groups. Digoxin immune Fab (DIF) treated EDLF positive, PE women had a delivery latency period 26 hours longer than placebo treated women, but this difference was not statistically significant (p=0.17). The rate of IVH in infants regardless of birth weight as well as the rate of IVH in infants with birth weights < 1250 grams were significantly lower in neonates of women receiving digoxin immune Fab (DIF) (p=0.015 for all infants and p=0.012 for infants < 1250 grams)

Table 3 compares maternal complications between the two study groups. The rate of maternal pulmonary edema was significantly lower in women receiving digoxin immune Fab (DIF) compared to those receiving placebo (p=0.035). Of note, no EDLF negative subject in this study experienced pulmonary edema, irrespective of treatment arm.

Evidence for increased levels of an EDLF in PE is substantial. Graves SW. Sodium regulation, sodium pump function and sodium pump inhibitors in uncomplicated pregnancy and preeclampsia. (Review) Frontiers in Bioscience 2007;12:2438- 2446. The recent clinical trial of digoxin immune Fab (DIF) in the treatment of severe PE provided additional support for EDLF participating in maternal features of the disease. Adair, et al. Amer J Perinatol 2010;27:655-662 However, not all women with PE appear to have detectable levels of EDLF and hence they would be very unlikely to benefit from digoxin immune Fab (DIF) treatment. An independent group recently reported 82% of preeclamptic women in their study had urinary EDLF levels (measured by antibody assay for marinobufagenin, a candidate EDLF) that exceeded levels found in normotensive pregnant women. Agunanne, et al. Am J Perinatol 2011;28:509-514. This level of EDLF positivity matches well the 78% observed in this study. The key findings of this analysis focusing on those subjects who were EDLF positive are: 1) The reductions of circulating EDLF compared to pretreatment levels in response to digoxin immune Fab (DIF) were of greater magnitude and more significant than the change observed in the original all patients study. Adair, et al. Amer J Perinatol 2010;27:655-662. 2) The effect of digoxin immune Fab (DIF) on the creatinine clearance in EDLF positive subjects was greater at each treatment time point as compared to EDLF positive subjects receiving placebo. 3) Among secondary maternal measures, when EDLF status was taken into account, it was found that digoxin immune Fab (DIF) treated PE women had significantly fewer occurrences of pulmonary edema compared with placebo, and 4) digoxin immune Fab (DIF) treatment in EDLF positive women was associated with significantly fewer cases of IVH, in all infants. We note that all IVH occurred in neonates with birth weight < 1250 grams.

The added suggestion that deterioration of renal function in untreated women was greater the greater the plasma EDLF level is a potentially novel finding. Previously, it has been presumed that diminished renal function gave rise to increased circulating EDLF levels, which may be true, but it is also true that EDLF has never been considered as a means by which renal function might be reduced. Agunanne et al. Am J Perinatol 2011;28:509-514.

ELDF has never been linked with pulmonary edema. However, high circulating EDLF has been associated with cerebral edema. Lusic, et al. Acta Neurochir 1999;141:691-697; Wijdicks, et al. Brit Med J 1987;294:729-733; Rap, et al. Acta Neurochir Suppl 1994;60:98-100. Additionally, in an animal model of PE, rats administered marinobufagenin, a sodium pump inhibitor and EDLF candidate, developed mesenteric post-capillary venules permeable to albumin. Uddin, et al. Am J Nephrol 2009;30:26-33. SP inhibition has also been studied as a way of reducing aqueous humor formation. Dismuke, et al. Brit J Ophthalmol 2009;93:104-109. The pumping of ions by the SP is accompanied by the movement of water molecules, which are tightly associated with the ions, across membranes. Given that 3 Na⁺ ions are moved out of cells for each 2 K⁺ ions transported in for each cycle of the pump, reduction in SP activity would likely be associated with water accumulation on the interior aspect of the cell membranes and potentially affect water retention in tissues.

While maternal measures other than CrCl and pulmonary edema did not demonstrate statistically significant improvements with digoxin immune Fab (DIF) treatment, the occurrence of several maternal abnormalities were reduced by more than half in the EDLF positive, digoxin immune Fab (DIF) treated group, collectively suggesting that digoxin immune Fab (DIF) may have exerted a positive effect in some of these women.

Digoxin immune Fab (DIF) treatment in EDLF positive women was associated with significantly fewer cases of IVH, in general and specifically in lower birth weight infants where IVH risk is greater. EDLF has not previously been associated with IVH. Consequently, its potential role in IVH and the possibility that digoxin immune Fab (DIF) protects the infant from IVH were not directly addressed by this study. Elevated EDLF levels are found in animals with intra-cerebroventricular hemorrhage, but clearly a more detailed assessment of EDLF's ability to cause or contribute to IVH is needed. Menezes et al., Amer J Hypertens 2003;16:1062-1065. Thus, ELDF may have a role in those processes that lead to IVH.

EDLF levels may be a response to more severe disease, but the finding of benefit in response to digoxin immune Fab (DIF) in only EDLF positive women makes it more likely that EDLF plays some pathogenic role in PE and its complications. It can also be stated that no fetal or neonatal parameter became significantly worse in the digoxin immune Fab (DIF)-treated, EDLF positive PE women as would be predicted if these were simply random artifacts.

As to how an EDLF might exert a fetal/neonatal effect, there is substantial evidence for the existence of circulating EDLF activity in the placenta, cord blood and fetal circulation immediately after birth but specific interactions have not been defined. Hopoate-Sitake et al. Reproductive Sci 2011;18:190-199; Morris, et al. Clin Sci 1987;73:291-297; Valdes, et al. J Pediatrics 1983; 102:947-950

In summary, the findings of this secondary analysis provide strong evidence that EDLF plays at least a contributory role in maternal aspects of PE and that digoxin immune Fab (DIF) treatment appears to ameliorate a number of maternal complications in women with severe preeclampsia provided that they are EDLF positive. These analyses in the EDLF positive subset of PE women raise the possibility of EDLF having a role in maternal pulmonary edema. The development of a rapid assay for EDLF, *i.e*. a theranostic, to determine who would benefit from therapy, would appear to be a useful assessment tool in any future study of digoxin immune Fab (DIF) treatment of PE women.

These findings also raise an interesting question about a possible role of EDLF in the occurrence of neonatal IVH and whether treatment with an anti-digoxin Fab might lower the incidence of this significant, life-threatening, neonatal complication. Therefore, an immunoassay for EDLF may be used to detect elevated levels of EDLF in patients carrying fetuses at risk for neonatal IVH and anti-digoxin antibody or antibody fragments thereof may be administered to treat or prevent the neonatal IVH.

**Table 1. Demographics of the EDLF Positive Subgroup**

| Parameter | Placebo n=19 | digoxin immune Fab (DIF) n=17 |
|---|---|---|
| Maternal age (yrs) | 25 + 5.1 | 26 ± 6.5 |
| Median parity (mean) | 1(1.2) | 1(0.9) |
| Race (% African American) | 7 (37) | 6 (35) |
| BMI (kg/m²) | 33 ± 6.4 | 35 ± 7.5 |
| MAP (mm Hg) | 111 ± 2.5 | 110 ± 1.9 |
| Gestational age at screen (d) | 202 ± 4.3 | 197 ± 4.7 |

No significant differences between groups in any of the analyzed variables.

**Table 2. Fetal/Neonatal outcomes according to treatment received.**

| Parameter | Placebo n=19 | digoxin immune Fab (DIF) n=17 | p-value |
|---|---|---|---|
| Latency period (hr) | 71 | 97 | 0.17 |
| Gestational age at delivery (days) | 205 ± 4.3 | 201 ± 4.4 | 0.53 |
| Birth weight (gm) | 1129.9 ± 92.8 | 974.2 ± 89.0 | 0.24 |
| Non-reassuring fetal status #(%) | 8 (42) | 4 (24) | 0.136 |
| Fetal heart rate abnormalities #(%) | 9 (47) | 4 (24) | 0.097 |
| Respiratory distress syndrome #(%) | 14 (74%) | 13 (76%) | 0.46 |
| IVH #(%) | 5(26) | 0 | 0.015 |
| Grade 3&4 #(%) | 3(16) | 0 | 0.053 |
| Infant < 1250 gm # (%) | 5/11 (42) | 0/14 | 0.012 |
| Neonatal death #(%) | 1(5.3) | 0 | 0.27 |

**Table 3. Maternal outcomes according to treatment received.**

| Parameter | Placebo n=19 | digoxin immune Fab (DIF) n=17 | p-value |
|---|---|---|---|
| Number completing 48 hour | 7 | 10 | 0.116 |
| treatment | | | |
| Blurred vision #(%) | 7 (37) | 3 (18) | 0.136 |
| Use of antihypertensives #(%) | 12 (63) | 7 (41) | 0.117 |
| HELLP syndrome #(%) | 1(5) | 1(6) | 0.512 |
| Pulmonary edema #(%) | 6 (32) | 1(6) | 0.035 |

### EXAMPLE 4

### Nanowire FET biosensors to detect EDLFs

A Silicon Nanowire Biosensor (SNB) system may be used for the detection of Endogenous Digoxin Like Factors (EDLFs). DigiFAB (Digoxin immune Fab) molecules were immobilized to the surface of the nanowires creating a EDLF biosensor. This biosensor could detect the binding of EDLFs in spiked buffer solution, compared to control buffer without EDLFs spiked. This SNB can be used for the successful and specific detection of EDLFs from spiked serum samples (100 nM- 10 µM). Using QuantuMDx Nanowire sensor technology a normalised average signal change of approximately 163% in the presence of EDLFs over the 4% average for non-spiked GoatFab and BSA control samples was observed. An observable response to the EDLFs was evident after a 4-hour incubation period in 88% of the FAB modified surfaces.

### Method

A simplified experimental approach was used to validate the electrical response of the sensor array to the presence of the EDLF. Employing characterisation of the initial device state a measureable change in the presence of the solvated EDLF (in this case, Digoxin) in direct comparison to experiments using biologically relevant protein models under controlled environmental conditions were defined. A multiplexed assay demonstrates the response of the electrical sensing system to various concentrations of Digoxin (100nM - 10 µM).

The nanowires were cleaned and activated using plasma asher. The oxide surface of the nanowires was chemically modified using APTES (1% solution in ethanol) producing an exposed NH₂ head group on the surface of the nanowires. Droplets (10 µL) of the appropriate protein (DigiFAB, anti-human IgG GoatFAB (hFAB), BSA, 1 mM) and coupling agents (DMAP, EDC, 10 µM) was deposited onto the chip surface. The reaction mixture in each instance was incubated overnight in a humid environment at room temperature. The chip was subsequently washed with excess 10xPBS buffer (performed 3 times) and again with deionised water (x3). Excess moisture was removed from the surface using a nitrogen stream and the chip was then placed in a vacuum oven to dry (20 min, 50°C). After drying, conductance of the nanowires was measured by sweeping through -5V to +5V using a two probe Agilent B1500 probe station. Upon completion of the initial conductance studies Digoxin spiked in buffer (1 µM in 0.5xSSC and 20 mM MgCl₂ buffer) was deposited onto the nanowire region of the chip and incubated for 4 hours at room temperature again in a humid environment. The chip was then thoroughly washed with excess 0.5x SSC buffer containing 20 mM MgCl₂ (x3), deionised H2O, dried with N₂ and then inserted into the vacuum oven for 20 minutes at 50°C to further dry. After drying, conductance of the nanowires was measured again by sweeping through -5V to +5V using a two probe Agilent B1500 probe station

### Results

The detection experiments were performed on QMDx SNB system using immobilized DigiFAB as the capture molecule. The electrical profile of the nanowire system was monitored pre and post Digoxin introduction (in controlled conditions) independently on DigiFAB, anti-human IgG-FAB (hFAB - a control protein of similar size to DigiFAB), and BSA modified nanowire surfaces.

Typically the measured nanowire response to the Digoxin on hFAB and BSA demonstrated a decrease in the conductivity profile of the sensing system in 100% of cases. Conversely when the Digoxin was introduced to the FAB the response of the system was opposite and offered an increase in the conductivity profile of the nanowire system in 63% of cases (minimally). *See* Fig 18A. Basic normalisation and signal processing when applied to the raw data convincingly delivers 100% positive response rate across the nanowire system.

**Table 4: Normalised average values and max / min values.**

| | Normalised Average | Max | Min |
|---|---|---|---|
| DiGi Fab | -0.65 | 8.86 | -0.92 |
| BSA | -0.96 | -0.90 | -0.99 |
| hFAB | -0.96 | -0.90 | -1.00 |

To test detection in a biological sample, digoxin was spiked into blood serum and the experiments repeated. The results again demonstrated the SNB ability to detect EDFLs, this time in a complex sample matrix. The specificity of the DiGiFAB to the Digoxin (and other EDLFs) suggests a degree of confidence in the ability and specificity of the technology to recognise the appropriate chemistry. An example of a FAB saturated surface is demonstrated in Fig. 18C, demonstrating the successful immobilization of DigiFAB on the silicon surface.

Thus, the system allows for a system to to detect EDLFs. Results with EDLF spiked blood serum samples support that the SNB is capable of detecting EDLFs from a biological sample. Therefore, this system may be applied to systems and methods for detecting EDLFs in blood which both concentrates the EDLFs and isolates them from the serum samples, using capture molecules immobilized on paramagnetic beads. This may allow for use of this system in methods for detecting EDLFs.

Those skilled in the art will recognize, or be able to ascertain using no more than routine experimentation, many equivalents to the specific embodiments of the invention described herein. Such equivalents are intended to be encompassed by the following claims.

### REFERENCES

Roberts JM. Preeclampsia: what we know and what we do not know. Semin Perinatal. 2000;24(1):24-28.
Roberts JM, Taylor RN, Musci TJ, Rodgers GM, Hubel CA, Mclaughlin MK. Preeclampsia: an endothelial cell disorder. Am J Obstet Gynecol. 1989; 161 (5): 1200-1204.
Zavalza-Gomez AB, Obesity and oxidative stress: a direct link to preeclampsia? Arch Gynecol Obstet. 2011;283:415-422.
Ness RB, Sibai BM. Shared and disparate components of the pathophysiologies of fetal growth restriction and preeclampsia, Am J Obstet Gynecol. 2006;195:40-49
LaMarca BD, Ryan MJ, Gilbert JS, Murphy SR, Granger JP. Inflammatory cytokines in the pathophysiology of hypertension during preeclampsia. Current Hypertension Reports. 2007; 9 (6): 480-485.
Sankaralingam S, Arenas IA, Lalu MM, Davidge ST. Preeclampsia: current understanding of the molecular basis of vascular dysfunction Expert Rev in Mol Med. 2006;8: 1-20
Granger JP, Alexander BT, Llinas MT, Bennett WA, Khalil RA. Pathophysiology of preeclampsia: linking placental ischemia/hypoxia with microvascular dysfunction. Microcirculation. 2002; 9 (3): 147-160.
Soleymanlou N, Jurisica I, Nevo O, Ietta F, Zhang X, Zamudio S. Molecular evidence of placental hypoxia in preeclampsia. J Clin Endocrinol Metab. 2005; 90 (7): 4299-4308. Graves SW, Williams GH. An endogenous ouabain-like factor associated with hypertensive pregnancies. J Clin Endocrinol Metab. 1984; 59:1070-4.
Valdes R Jr, Graves SW, Knight AB, Craig HR. Endogenous digoxin-immunoactivity is elevated in hypertensive pregnancy. Prog Clin Biol Res. 1985; 192:229-32.
Graves SW. The possible role of digitalis-like factors in pregnancy-induced hypertension. Hypertens. 1987; 10(S Pt 2):I-84-6.
Graves SW, Lincoln K, Cook SL, Seely EW. Digitalis-like factor and digoxin-like immunoreactive factor in diabetic women with preeclampsia, transient hypertension of pregnancy, and normotensive pregnancy. Am J Hypertens. 1995; 8:5-11.
Hamlyn JM, Blaustein MP, Bova S, DuCharme DW, Harris DW, Mandel F, Mathews WR, Ludens JH. Identification and characterization of a ouabain-like compound from human plasma. Proc Natl Acad Sci USA. 1991; 88: 6259-6263.
Goto, A. and Yamada K. Purification of endogenous digitalis-like factors from normal human urine. Clin Exp Hypertens._1998; 20: 551-56.
Glatter KA, Graves SW, Hollenberg NK, Soszynski PA, Tao QF, Frem GJ, Williams GH, Lazarus JM. Sustained volume expansion and [Na,K]ATPase inhibition in chronic renal failure. Am J Hypertens, 1994; 7:1016-25.
Krep HH, Price DA, Soszynski P, Tao Q-F, Graves SW, Hollenberg NK. Volume sensitive hypertension and the digoxin-like factor: reversal by an Fab directed against digoxin in DOCA:salt hypertensive rats. Am J Hypertens. 1995; 8:921-7.
Krep HH, Graves SW, Price DA, Lazarus M, Ensign A, Soszynski PA, Hollenberg NK. Reversal of sodium pump inhibitor induced vascular smooth muscle contraction with Digibind: stoichiometry and its implications. Am J Hypertens. 1995; 9:39-46.
Smith TW, Butler VP Jr, Haber E, Fozzard H, Marcus FI, Bremner WF, Schulman IC, Phillips A. Treatment of life-threatening digitalis intoxication with digoxin-specific Fab antibody fragments: experience in 26 cases. N Engl J Med. 1982;307:1357-62.
Adair CD, Buckalew VM, Graves SW, Lam GK, Johnson DD, Saade G, Lewis DF, Robinson C, Danoff TM, Chauhan N, Hopoate-Sitake M, Porter KB, Humphrey RG, Trofatter KF, Amon E, Ward S, Kennedy L, Mason L, Johnston JA. Digoxin immune Fab treatment for severe preeclampsia. Amer J Perinatol. 2010;27:655-662.
Loose DS, Kan PB, Hirst MA, Marcus RA and Feldman D. Ketoconazole blocks adrenal steroidogenesis by inhibiting cytochrome P-450 dependent enzymes. J. Clin. Invest. 1983;71: 1495-1499.
Miossec P, Archambeaud-Mouveroux F and Teissier MP. Inhibition of steroidogenesis by ketoconazole. Therapeutic uses. Ann Endocrinol (Paris). 1997:58: 494-502.
Kraemer FB, Spilman SD. Effect of ketoconazole on cholesterol synthesis. J Pharmacol Exp Ther. 1986;238:905-911.
Kempen HJ, Van son K, Cohen LH, Griffioen M. Effect of ketoconazole on cholesterol synthesis and on HMG-CoA reductase and LDL-receptor activities in Hep G2 cells. Biochem Pharmacol. 1987;36:1245-1249.
Shibata Y, Takahashi H, Chiba M, Ishii Y. A novel approach to the prediction of drug-drug interactions in humans based on the serum incubation method. Drug Metab Pharmacokinet. 2008; 23:328-339.
Levy MJ, Smotrich DB, Widra EA, Sagoskin AW, Murray DL, Hall JL. The predictive value of serum progesterone and 17-OH progesterone levels on in vitro fertilization outcome. Amer Fertility Soc. 1995;12:161-166.
Li H, Gu B, Zhang Y, Lewis DF, Wang Y. Hypoxia-induced increase in soluble Flt-1 production correlates with enhanced oxidative stress in trophoblast cells from the human placenta. Placenta. 2005;26:210-217.
Hopoate-Sitake ML, Adair CD, Mason LA, Torres C, Kipikasa J, Graves SW. Digibind reverses inhibition of cellular Rb+ uptake caused by endogenous sodium pump inhibitors present in serum and placenta of women with preeclampsia. Reproductive Sci. 2011;18:190-199.
Diamandis EP, Papanastaslou-Diamandi A, Soldin SJ. Digoxin immunoreactivity in cord and maternal serum and placental extracts. Partial characterization of immunoreactive substances by high-performance liquid chromatography and inhibition of Na+,K+-ATPase. Clin Biochem. 1985;18:48-55.
Morris JF, Poston L, Wolfe CD, Hilton PJ. A comparison of endogenous digoxin-like immunoactivity and sodium transport inhibitory activity in umbilical arterial and venous serum. Clin Sci (Lond). 1988;75:577-579.
Hilton PJ, White RW, Lord GA, Garner GV, Gordon DB, Hilton MJ, Forni LG, McKinnon W, Ismail RMD, Keenan M, Jones K, Morden WE. An inhibitor of the sodium pump obtained from human placenta. Lancet 1996;348:303-305.
Gao S, Chen Z, Xu Y. The source of endogenous digitalis-like substance in normal pregnancy. Zhonghua Fu Chan Ke Za Zhi. 1998;33:539-541.
Fedorova OV, Tapilskaya NI, Bzhelyansky AM, Frolova EV, Nikitina ER, Reznik VA, Kashkin VA, Bagrov AY. Interaction of Digibind with endogenous cardiotonic steroids from preeclamptic placentae. J Hypertens. 2010;28:361-366.
Ugele B, Simon S. Uptake of dehydroepiandrosterone-3sulfate by isolated trophoblasts from human term placenta, JEG-3, BeWo, Jar, BHK cells and BHK cells transfected with human sterylsulfatase-cDNA. J Steroid Biochem Mol Biol. 1999;71:203-211.
Fang S, Shoko H, Yukako O, Sakiko H, Kyoko T, Yasuko Y, Sadako T and Shosuke K. Evaluation of oxidative stress based on lipid hydroperoxide, vitamin C and vitamin E during apoptosis and necrosis caused by thioacetamide in rat liver. BBA-Molec Basis Disease. 2000;1500:181-185.
Rabe T, Brandstetter K, Kellermann J, Runnebaum B. Partial characterization of placental 3 beta-hydroxysteroid dehydrogenase (EC1.1.1.145), delta 4-5 isomerase (EC 5.3.3.1) in human term placenta. J Steroid Biochem. 1982;17:427-433.
Powell WA, Challis JR, Influence of 20 alpha-dihydroprogesterone on progesterone output by human chorion explants. Gynecol Obstet Invest. 1986;22:73-78.
Goto A, Yamada K, Yagi N, Yoshioka M, Sugimoto T. Physiology and pharmacology of endogenous digitalis-like factors. Pharmacol Rev 1992;44:377-99.
Haddy FJ, Buckalew VM Jr. Endogenous digitalis-like factors in hypertension. In Brenner BM and Laragh JH (eds) Hypertension: Pathophysiology, Diagnosis, and Management. Raven Press, New York, 1995; pp 1055-1067.
Graves SW, Williams GH. Endogenous digitalis-like natriuretic factors. Ann Rev Med 1987;38:433-444.
Graves SW. The sodium pump in hypertension. Curr Opinion Nephrol Hypertens 1994;3:107-111.
Graves SW, Williams GH. An endogenous ouabain-like factor associated with hypertensive pregnancies. J Clin Endocrinol Metab 1984; 59:1070-4.
Graves SW. The possible role of digitalis-like factors in pregnancy-induced hypertension. Hypertension 1987; 10(S Pt 2):I-84-6.
Graves SW. Sodium regulation, sodium pump function and sodium pump inhibitors in uncomplicated pregnancy and preeclampsia. Frontiers in Bioscience 2007;12:2438-2446. Smith, et al. Treatment of life-threatening digitalis intoxication with digoxin-specific Fab antibody fragments: experience in 26 cases. N Engl J Med 1982;307:1357-62.
Krep HH, Graves SW, Price DA, Lazarus M, Ensign A, Soszynski PA, Hollenberg NK. Reversal of sodium pump inhibitor induced vascular smooth muscle contraction with Digibind: stoichiometry and its implications. Am J Hypertens 1995; 9:39-46.
Goodlin RC: Antidigoxin antibodies in eclampsia. N Eng J Med 1988;318:518-519. Adair DA, Hinshaw G, Russell J, Rose J, Veille J-C, Buckalew V. Effects of Fab digoxin-specific antibodies on mean arterial pressure in severe preeclampsia. Am J Hypertens 1997;10:11A
Adair CD, Buckalew VM, Taylor K, Ernest JM, Frye AH, Evans C, Veille J-C. Elevated endoxin-like factor complicating a multifetal second trimester pregnancy: treatment with digoxin-binding immunoglobulin. Am J Nephrol 1996;16:529-531
Adair CD, Luper A, Rose JC, Russell G, Veille JC, Buckalew VM. The hemodynamic effects of intravenous digoxin-binding Fab immunoglobulin in severe preeclampsia: a double-blind, randomized, clinical trial. J Perinatol 2009;29:284-9.
Barton JR, Sibai BM. Prediction and prevention of recurrent preeclampsia. Obstet Gynecol 2008;112:359-372.
Sibai BM, Barton JR. Expectant management of severe preeclampsia remote from term: patient selection, treatment, and delivery indications. Am J Obstet Gynecol 2007; 196:514.e1-514.e9.
Krep HH, Graves SW, Price DA, Lazarus M, Ensign A, Soszynski PA, Hollenberg NK. Reversal of sodium pump inhibitor induced vascular smooth muscle contraction with Digibind: stoichiometry and its implications. Am J Hypertens 1995; 9:39-46.
Soszynski PA, Ensign A, Graves SW, Hollenberg NK. Specificity of the vascular smooth muscle contractile response to a labile digitalis-like factor in peritoneal dialysate: The influence of potassium. Am J Hypertens 1997;10:1342-48.
Krep H, Price DA, Soszynski P, Tao Q-F, Graves SW, Hollenberg NK. Volume sensitive hypertension and the digoxin-like factor: reversal by an Fab directed against digoxin in DOCA:salt hypertensive rats. Am J Hypertens 1995;8:921-7.
Glatter KA, Graves SW, Hollenberg NK, Soszynski PA, Tao QF, Frem GJ, Williams GH, Lazarus JM. Sustained volume expansion and [Na,K]ATPase inhibition in chronic renal failure. Am J Hypertens 1994;7:1016-25.
Graves SW, Williams GH. An endogenous ouabain-like factor associated with hypertensive pregnancies. J Clin Endocrinol Metab 1984;59:1070-4.
Gusdon JP, Buckalew VM Jr, Hennessy JF. A digoxin-like immunoreactive substance in preeclampsia. Am J Obstet Gynecol 984;15:83-85.
Seely EW, Williams GH, Graves SW. Markers of sodium and volume homeostasis in pregnancy-induced hypertension. J Clin Endocrinol Metab 1992; 74:150-6.
Lopatin DA, Ailamazian EK, Dmitrieva RI, Shpen VM, Fedorova OV, Doris PA, Bagrov AY. Circulating bufodienolide and cardenolide sodium pump inhibitors in preeclampsia. J Hypertens 1999;17:1179-1187.
Hopoate-Sitake ML, Adair CD, Mason LA, Torres C, Kipikasa J, Graves SW. Digibind reverses inhibition of cellular Rb+ uptake caused by endogenous sodium pump inhibitors present in serum and placenta of women with preeclampsia. Reproductive Sci 2011;18:190-199.
Goodlin RC. Antidigoxin antibodies in eclampsia. N Engl J Med 1988; 318:518-9. Adair CD, Backalew VM, Taylor K et al. Elevated endoxin-like factor complicating a second trimester pregnancy: treatment with digoxin-binding immunoglobin. Am J Nephrol 1996;6(6):529-31.
Adair CD, Luper A, Rose JC, Russell G, Veille J-C, Buckalew VM. The hemodynamic effects of intravenous digoxin-binding fab immunoglobulin in severe preeclampsia: a double-blind randomized, clinical trial. J Perinatolog 2009;29:284-289.
Adair CD, Buckalew VM, Graves SW, Lam GK, Johnson DD, Saade G, Lewis DF, Robinson C, Danoff TM, Chauhan N, Hopoate-Sitake M, on behalf of the DEEP Study Group. Digoxin Immune Fab Treatment for Severe Preeclampsia. Amer J Perinatol 2010;27:655-662.
Zhen Y, Franz KB, Graves SW. A novel assay of cell rubidium uptake using graphite furnace atomic absorption: Application to rats on a magnesium deficient diet. J Nutritional Biochem 2005;16:291-296.
Mehta CR, Hilton JF. Exact power of conditional and unconditional tests: going beyond the 2x2 contingency table. Amer Statistician 1993;47:91-98.
Chan I. Exact tests of equivalence and efficacy with a non-zero lower bound for comparative studies. Statistics Med. 1998;17:1403-1413.
Graves SW. Sodium regulation, sodium pump function and sodium pump inhibitors in uncomplicated pregnancy and preeclampsia. (Review) Frontiers in Bioscience 2007;12:2438- 2446.
Agunanne E, Horvat D, Harrison R, Uddin MN, Jones R, Kuehl TJ, Ghanem DA, Berghman LR, Lai X, Li J, Romo D, Puschett JB. Marinobufagenin levels in preeclamptic patients: A preliminary report. Am J Perinatol 2011;28:509-514.
Graves SW, Glatter K, Lazarus MJ, Hollenberg NK. Volume expansion in renal failure patients: a paradigm for a clinically relevant [Na,K]ATPase inhibitor. J Cardiovasc Pharmacol 1993; 22(S2):S54-57.
Lusic I, Ljutic D, Maskovic J, Jankovic S. Plasma and cerebrospinal fluid endogenous digoxin-like immunoreactivity in patients with aneurismal subarachnoid hemorrhage. Acta Neurochir 1999;141:691-697.
Wijdicks EFM, Vermeulen M, Van Brummelen P, Den Boer NC, Van Gijn J. Digoxin-like immunoreactive substance in patients with aneurismal subarachnoid hemorrhage. Brit Med J 1987;294:729-733.
Rap ZM, Schoner W, Czernicki Z, Hildebrandt G, Mueller HW, Hoffmann O. The endogenous ouabain-like sodium pump inhibitor in cold injury-induced brain edema. Acta Neurochir Suppl 1994;60:98-100.
Uddin MN, McLean LB, Hunter FA, Horvat D, Severson J, Tharakan B, Childs EW, Puschett JB. Vascular leak in a rat model of preeclampsia. Am J Nephrol 2009;30:26-33. Dismuke WM, Mbadugha CC Faison D, Ellis DZ. Ouabain-induced changes in aqueous humour outflow facility and trabecular meshwork sytoskeleton. Brit J Ophthalmol 2009;93: 104-109.
Menezes JC, Dichtchekenian V. Digoxin antibody prevents cerebral hemorrhage-induced hypertension. Amer J Hypertens 2003;16:1062-1065.
Morris JF, McEachern MD, Poston L, Smith SE, Mulvany MJ, Hilton PJ. Evidence for an inhibitor of leucocyte sodium transport in the serum of neonates. Clin Sci 1987;73:291-297.
Valdes R Jr, Graves SW, Brown BA, Landt M. Endogenous substances in newborn infants causing false-positive digoxin measurements. J Pediatrics 1983; 102:947-950.

Further embodiments are set out in the below numbered clauses.
Clause 1. A method of administering digoxin immune Fab (DIF) to treat eclampsia or preeclampsia comprising: (a) conducting a digoxin-immune antibody assay on a patient suffering from eclampsia or preeclampsia; (b) determining whether the patient is EDLF positive based on the assay; and (c) administering digoxin immune Fab (DIF) to patient if the patient is determined to be EDLF positive.
Clause 2. A method of administering digoxin immune Fab (DIF) to treat a gravid human patient exhibiting at least one symptom of gestational hypertension, preeclampsia, eclampsia, or intrauterine growth restriction comprising: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive. Clause 3. A method of treating a gravid human patient exhibiting at least one symptom of gestational hypertension, preeclampsia, eclampsia, or intrauterine growth restriction comprising: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.
Clause 4. A method of administering digoxin immune Fab (DIF) to a patient to prevent intraventricular hemorrhage (IVH) in the neonate of the patient comprising: (a) conducting a digoxin-immune antibody assay on the patient; (b) determining whether the patient is EDLF positive based on assay; and (c) administering digoxin immune Fab (DIF) to patient if the patient is determined to be EDLF positive.
Clause 5. A method of administering digoxin immune Fab (DIF) to treat intraventricular hemorrhage comprising: (a) conducting a digoxin-immune antibody assay on a gravid human patient whose fetus may develop IVH as a result of being delivered prematurely (before 40 weeks gestation); (b) determining whether the patient is EDLF positive based on assay; and (c) administering digoxin immune Fab (DIF) to patient if the patient is determined to be EDLF positive.
Clause 6. A method of administering an anti-digoxin antibody or antigen-binding fragment thereof, optionally a Digoxin immune Fab, to prevent intraventricular hemorrhage (IVH) in the neonate of the patient comprising: (a) conducting a digoxin-immune antibody assay on a gravid human patient whose fetus may develop IVH as a result of being delivered prematurely (before 40 weeks gestation); (b) determining whether the patient is EDLF positive based on assay; and (c) administering the anti-digoxin antibody or antigen-binding fragment thereof to patient if the patient is determined to be EDLF positive.
Clause 7. A method of administering an anti-digoxin antibody or antigen-binding fragment thereof to treat fetal complications associated with premature birth, including comprising: (a) conducting a digoxin-immune antibody assay on a gravid human patient whose fetus may be delivered prematurely (before 40 weeks gestation); (b) determining whether the patient is EDLF positive based on assay; and (c) administering the anti-digoxin antibody or antigen-binding fragment thereof to patient if the patient is determined to be EDLF positive.
Clause 8. The method of clause 7, wherein the fetal complications associated with premature birth include IVH or NEC.
Clause 9. A method of extending pregnancy in a gravid human patient exhibiting at least one symptom of gestational hypertension, preeclampsia, eclampsia, or intrauterine growth restriction comprising: (a) conducting a digoxin-immune antibody assay on a patient suffering from eclampsia or preeclampsia; (b) determining whether the patient is EDLF positive based on assay; and (c) administering digoxin immune Fab (DIF) to patient if the patient is determined to be EDLF positive.
Clause 10. A method for treating a patient at risk for eclampsia or preeclampsia comprising: (a) obtaining a sample from a patient at risk for eclampsia or preeclampsia; (b) contacting said sample with an anti-EDLF antibody or antibody fragment thereof; (c) detecting the presence of an anti-EDLF antibody or antibody fragment-EDLF complex, wherein the presence of said EDLF is indicative of eclampsia or preeclampsia; and (d) administering digoxin immune Fab (DIF) to patient if the patient is determined to be EDLF positive.
Clause 11. A method for treating a patient whose fetus and/or neonate is at risk for IVH comprising: (a) obtaining a sample from a patient; (b) contacting said sample with an anti-EDLF antibody or antibody fragment thereof; (c) detecting the presence of an anti-EDLF antibody or antibody fragment-EDLF complex, wherein the presence of said EDLF is indicative of eclampsia or preeclampsia; and (d) administering digoxin immune Fab (DIF) to patient if the patient is determined to be EDLF positive.
Clause 12. A method for screening patients for responsiveness to anti-digoxin therapy for eclampsia or preeclampsia: (a) obtaining a sample from a patient at risk for eclampsia or preeclampsia (b) assaying for the presence of EDLF; (c) determining the EDLF level; (d) administering an anti-digoxin antibody or antibody fragment thereof, , optionally a Digoxin immune Fab, to said patient if said EDLF level is over 100 nm EDLF.
Clause 13. A method for screening patients for responsiveness to anti-digoxin therapy for gestational hypertension, preeclampsia, eclampsia, or intrauterine growth restriction comprising: (a) obtaining a sample from a patient suffering from gestational hypertension, preeclampsia, eclampsia, or intrauterine growth restriction; (b) assaying for the presence of EDLF; (c) administering an anti-digoxin antibody or antibody fragment thereof, optionally a Digoxin immune Fab, to said patient if the patient is determined to be EDLF positive.
Clause 14. A method for screening patients for responsiveness to anti-digoxin therapy for gestational hypertension, preeclampsia, eclampsia, or intrauterine growth restriction comprising: (a) obtaining a sample from a patient at risk for gestational hypertension, preeclampsia, eclampsia, or intrauterine growth restriction; (b) assaying for the presence of EDLF; (c) administering an anti-digoxin antibody or antibody fragment thereof, optionally a Digoxin immune Fab, to said patient if the patient is determined to be EDLF positive.
Clause 15. A method for screening patients for responsiveness to anti-digoxin therapy for gestational hypertension, preeclampsia, eclampsia, or intrauterine growth restriction comprising: (a) obtaining a sample from a patient at risk for gestational hypertension, preeclampsia, eclampsia, or intrauterine growth restriction; (b) assaying for the presence of EDLF; (c) determining the EDLF level; (d) administering an anti-digoxin antibody or antibody fragment thereof to said patient if said EDLF level is over 100 nm EDLF. Clause 16. A method of administering anti-digoxin antibody or antigen-binding fragment thereof to treat intraventricular hemorrhage comprising: (a) conducting a digoxin-immune antibody radioimmunoassay on a patient suffering from intraventricular hemorrhage; (b) determining whether the patient is EDLF positive based on radioimmunoassay; and (c) administering the anti-digoxin antibody or antigen-binding fragment thereof to patient if the patient is determined to be EDLF positive.
Clause 17. A method for treating intraventricular hemorrhage comprising: (a) obtaining a sample from a patient whose fetus is at risk for intraventricular hemorrhage (b) assaying for the presence of EDLF; (c) determining the EDLF level; (d) administering an anti-digoxin antibody or antibody fragment thereof to said patient if said EDLF level is over 100 nm EDLF.
Clause 18. A method for screening patients for responsiveness to anti-digoxin therapy for intraventricular hemorrhage: (a) obtaining a sample from a patient at risk for intraventricular hemorrhage (b) assaying for the presence of EDLF; (c) determining the EDLF level; (d) administering an anti-digoxin antibody or antibody fragment thereof, optionally a Digoxin immune Fab, to said patient if said EDLF level is over 100 nm EDLF.
Clause 19. A method for treating a patient at risk for intraventricular hemorrhage comprising: (a) obtaining a sample from a patient at risk for intraventricular hemorrhage; (b) contacting said sample with an anti-EDLF antibody or antibody fragment thereof; and (c) detecting the presence of an anti-EDLF antibody or antibody fragment-EDLF complex, wherein the presence of said EDLF is indicative of intraventricular hemorrhage. Clause 20. The method of any one of clauses 6, 7, 12, 13, or 15-18, wherein said fragment is a Fab, Fab', F(ab')2, Fv, CDR, paratope, or portion of an antibody that is capable of binding the antigen.
Clause 21. The method of any one of clauses 6, 7, 12, 13, or 15-18, wherein said antibody is chimeric, humanized, anti-idiotypic, single-chain, bifunctional, or co-specific. Clause 22. The method of any one of clauses 6, 7, 12, 13, or 15-18, wherein said antibody or fragment is conjugated to a label.
Clause 23. The method of clause 22, wherein said label is a chemiluminescent label, paramagnetic label, an MRI contrast agent, fluorescent label, bioluminescent label, or radioactive label.
Clause 24. The method of clause 22, wherein said paramagnetic label is aluminum, manganese, platinum, oxygen, lanthanum, lutetium, scandium, yttrium, or gallium. Clause 25. The method of any one of clauses 6, 7, 12, 13, or 15-18, wherein said antibody is attached to a solid support.
Clause 26. The method of clause 25, wherein said solid phase support is a bead, test tube, sheet, culture dish, nanowire or test strip.
Clause 27. The method of clause 25, wherein said solid support is an array.
Clause 28. The method of any one of clauses 10-15 or 17-19, wherein said sample is a blood, serum, plasma, or placenta sample.
Clause 29. The method of any one of clauses 1-7, 9, or 16, wherein the antibody assay includes an assay selected from the group consisting of Western blots, adioimmunoassays, ELISA (enzyme linked immunosorbent assay), "sandwich" immunoassays, immunoprecipitation assays, precipitation reactions, gel diffusion precipitation reactions, immunodiffusion assays, agglutination assays, complement-fixation assays, immunohistochemical assays, fluorescent immunoassays, and protein A immunoassays.
Clause 30. The method of any one of clauses 12, 15, 17, or 18, wherein said EDLF level is over 100 nM EDLF.
Clause 31. The method of any one of clauses 10-15 or 17-19, wherein the administered dosage of digoxin antibody is at least than 0.006 mg digoxin binding capacity /Kg.
Clause 32. The method of any one of clauses 10-15 or 17-19, wherein the dosage is administered over a period of six hours or less.
Clause 33. The method of any one of clauses 10-15 or 17-19, further comprising administration of subsequent dosages of digoxin immune Fab.
Clause 34. The method of any one of clauses 10-15 or 17-19, further comprising administering a therapeutically effective amount of corticosteroid.
Clause 35. The method of any one of clauses 10-15 or 17-19, further comprising administration of subsequent dosages of digoxin immune Fab.
Clause 36. The method of any one of clauses 10-15 or 17-19, wherein the method further comprises administering a therapeutically effective amount of an antihypertensive drug.
Clause 37. The method of clause 36, wherein the antihypertensive drug is labetalol, altenolol, nifedipine, 1-methyldopa or hydralazine.
Clause 38. The method of any one of clauses 10-15 or 17-19, wherein the method further comprises administering a therapeutically effective amount of magnesium sulfate or phenytoin.
Clause 39. The method of any one of clauses 10-15 or 17-19, wherein the digoxin immune Fab is ovine digoxin immune Fab.
Clause 40. The method of any one of clauses 10-15 or 17-19, wherein the dose is no more than approximately 10.0 mg.
Clause 41. The method of any one of clauses 10-15 or 17-19, wherein the dose is no more than approximately 5.0 mg.
Clause 42. The method of any one of clauses 10-15 or 17-19, wherein the dose is in the range between approximately 0.01 to 1.0 mg.
Clause 43. The method of any one of clauses 10-15 or 17-19, wherein the dose is in the range between approximately 0.01 mg to 0.5 mg.
Clause 44. A silicon nanowire biosensor comprising an immobilized anti-digoxin antibody, optionally an anti-digoxin Fab antibody fragment.
Clause 45. The biosensor of clause 44, wherein said fragment is a Fab, Fab', F(ab')2, Fv, CDR, paratope, or portion of an antibody that is capable of binding the antigen, optionally a Digoxin immune Fab.
Clause 46. The biosensor of clause 45, wherein said antibody is chimeric, humanized, anti-idiotypic, single-chain, bifunctional, or co-specific.
Clause 47. The biosensor of clause 46, wherein said biosensor has a sensativity of at least about 100 nM of digoxin in a biological sample.
Clause 48. A method of detecting EDLF comprising contacting a biological sample with the biosensor of clause 44 and assaying for the presence of EDLF.

## Claims

1. An anti-digoxin antibody or antigen-binding fragment thereof for use in the treatment or prevention of intraventricular hemorrhage (IVH) in the neonate of an EDLF positive gravid human patient, wherein the patient's EDLF level is determined to be over 100 nM EDLF.

2. An anti-digoxin antibody or antigen-binding fragment thereof for use in the treatment or prevention of intraventricular hemorrhage (IVH) in an EDLF positive neonate human patient, wherein the patient's EDLF level is determined to be over 100 nM EDLF.

3. A method for screening patients for responsiveness to anti-digoxin therapy for intraventricular hemorrhage:
(a) assaying a sample obtained from a gravid human patient at risk for having a neonate with intraventricular hemorrhage for the presence of EDLF using an antibody or antigen-binding fragment;
(b) determining the EDLF level;
wherein an EDLF level over 100 nM EDLF is indicative of responsiveness to anti-digoxin therapy for intraventricular hemorrhage.

4. A method for diagnosing a patient at risk for intraventricular hemorrhage comprising:
(a) contacting a sample obtained from a patient at risk for intraventricular hemorrhage with an anti-EDLF antibody or antibody fragment thereof; and
(b) detecting the presence of an anti-EDLF antibody or antibody fragment-EDLF complex,
wherein the presence of an EDLF level over 100 nM EDLF is indicative of intraventricular hemorrhage.

5. The method claim 3 or 4, wherein said fragment is a Fab, Fab', F(ab')2, Fv, CDR, paratope, or portion of an antibody that is capable of binding the antigen, or wherein said antibody is chimeric, humanized, anti-idiotypic, single-chain, bifunctional, or co-specific, or wherein said antibody or fragment is conjugated to a label.

6. The method of claim 5, wherein said label is a chemiluminescent label, paramagnetic label, an MRI contrast agent, fluorescent label, bioluminescent label, or radioactive label, or wherein said paramagnetic label is aluminum, manganese, platinum, oxygen, lanthanum, lutetium, scandium, yttrium, or gallium.

7. The method of claim 3 or 4, wherein said antibody is attached to a solid support, preferably wherein said solid phase support is a bead, test tube, sheet, culture dish, nanowire or test strip or wherein said solid support is an array.

8. The method of claim 3 or 4, wherein said sample is a blood, serum, plasma, or placenta sample.

9. The method of claim 3 or 4, wherein the assay is selected from the group consisting of Western blots, radioimmunoassays, ELISA (enzyme linked immunosorbent assay), "sandwich" immunoassays, immunoprecipitation assays, precipitation reactions, gel diffusion precipitation reactions, immunodiffusion assays, agglutination assays, complement- fixation assays, immunohistochemical assays, fluorescent immunoassays, and protein A immunoassays.

10. The anti-digoxin antibody or antigen-binding fragment thereof for use according to claim 1 or 2, wherein the digoxin antibody is to be administered at a dosage of at least than 0.006 mg digoxin binding capacity /Kg; or wherein the dosage is administered over a period of six hours or less; or wherein subsequent dosages of digoxin immune Fab are to be administered.

11. The anti-digoxin antibody or antigen-binding fragment thereof for use according to claim 1 or 2, further wherein a therapeutically effective amount of a corticosteroid, magnesium sulfate, phenytoin, and/or an antihypertensive drug is to be administered, preferably wherein the antihypertensive drug is labetalol, altenolol, nifedipine, 1-methyldopa, or hydralazine.

12. The anti-digoxin antibody or antigen-binding fragment thereof for use according to claim 1 or 2, wherein the dose is no more than approximately 10.0 mg; or wherein the dose is no more than approximately 5.0 mg; or wherein the dose is in the range between approximately 0.01 to 1.0 mg; or wherein the dose is in the range between approximately 0.01 mg to 0.5 mg.

13. The method of claim 3, wherein determining the EDLF level comprises contacting the sample with a silicon nanowire biosensor comprising an immobilized anti-digoxin antibody.
